Europäisches Patentamt
European Patent Office
Office européen des brevets

(19) (11) Veröffentlichungsnummer: **0 193 083**
**A1**

(12) # EUROPÄISCHE PATENTANMELDUNG

(21) Anmeldenummer: **86102092.3**

(22) Anmeldetag: **18.02.86**

(51) Int. Cl.[4]: **C 07 F 15/00**
A 61 K 31/28

(30) Priorität: **23.02.85 DE 3506468**

(43) Veröffentlichungstag der Anmeldung:
**03.09.86 Patentblatt 86/36**

(84) Benannte Vertragsstaaten:
**AT BE CH DE FR GB IT LI LU NL SE**

(71) Anmelder: **Asta-Werke Aktiengesellschaft Chemische Fabrik**
**Artur-Ladebeck-Strasse 128 - 152**
**D-4800 Bielefeld 14(DE)**

(72) Erfinder: **Brunner, Henri, Prof.**
**Eichendorffstrasse 14**
**D-8411 Lappersdorf(DE)**

(72) Erfinder: **Schönenberger, Helmut, Prof.**
**Ahornstrasse 14**
**D-8401 Pentling(DE)**

(72) Erfinder: **Schmidt, Manfred, Dr.**
**Unter-Haitzergasse 19**
**D-6460 Gelnhausen(DE)**

(72) Erfinder: **Holzinger, Ulrich**
**Neuburger Strasse 102**
**D-8390 Passau(DE)**

(72) Erfinder: **Unger, Gerfried, Dr.**
**Mittelweg 50**
**D-6000 Frankfurt/Main 1(DE)**

(72) Erfinder: **Engel, Jürgen, Dr.**
**Erlenweg 3**
**D-8755 Alzenau(DE)**

(54) **Tumorhemmende (1-Benzyl-ethylendiamin)-platin(II)-Komplexe.**

(57) Tumorhemmende (1-Benzyl-ethylendiamin)-platin (II)-Komplexe der allgemeinen Formel

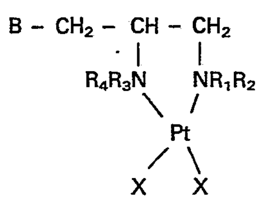

I

worin die Reste $R_1$, $R_2$, $R_3$ und $R_4$ gleich oder verschieden sind und Wasserstoff, eine $C_1$-$C_6$-Alkylgruppe, eine Benzylgruppe oder eine Phenylethylgruppe bedeuten, und B ein Thienylrest, ein Indolylrest, ein Imidazolylrest oder ein durch die Reste $R_5$, $R_6$ und $R_7$ substituierter Phenylrest ist und die Reste $R_5$, $R_6$ und $R_7$ gleich oder verschieden sind und Wasserstoff, Halogen, Trihalogenmethyl, $C_1$–$C_6$–Alkyl, Hydroxy, $C_1$–$C_6$–Alkoxy, Phenoxy, Benzyloxy, $C_1$–$C_6$–Alkanoyloxy, Benzoyloxy, $C_1$–$C_6$–Alkansulfonyloxy, Carboxy, $C_1$–$C_6$–Carbalkoxy, Cyano, Aminocarbonyl, Aminocarbonyl, welches einen oder zwei $C_1$–$C_6$–Alkylreste enthält, $C_1$–$C_6$–Alkylcarbonyl, Nitro, Amino, $C_1$–$C_6$–Alkylamino, Di–$C_1$–$C_6$–alkylamino, $(C_1$–$C_6$–$Alkyl)_3N^{\oplus}$, $C_1$–$C_6$–Alkanoylamino, $C_1$–$C_6$–Alkyl–$C_1$–$C_6$–alkanoylamino, $C_1$–$C_6$–Alkansulfonylamino, $C_1$–$C_6$–Alkyl–$C_1$–$C_6$–alkansulfonylamino, Aminosulfonyl, Aminosulfonyl, welches einen oder zwei $C_1$–$C_6$–Alkylreste enthält, $C_1$–$C_6$–Alkoxysulfonyl ($-SO_2-O-C_1-C_6$–Alkyl), Sulfo ($-SO_3H$) oder $C_1$–$C_6$–Alkansulfonyl bedeuten und zwei dieser Reste auch die Methylendioxygruppe sein können und X für das Äquivalent eines physiologisch verträglichen Anions steht,
sowie gegebenenfalls deren Salze mit physiologisch verträglichen Kationen oder Anionen und deren Herstellung.

EP 0 193 083 A1

0193083

Degussa Aktiengesellschaft
Weissfrauenstraße 9, 6000 Frankfurt/Main

# Tumorhemmende (1-Benzyl-ethylendiamin)-platin(II)-Komplexe

Es ist bekannt, daß Verbindungen der folgenden Formel

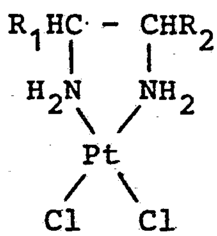

$R_1$ = H, Alkyl
$R_2$ = H, Alkyl

eine Antitumorwirkung besitzen (Journal of Inorganic Biochemistry 11, 1979, Seiten 139 - 149; Biochimie 60, 1978, Seiten 835 - 850). Weiterhin sind aus der deutschen Offenlegungsschrift 34 05 611 antitumorwirksame Verbindungen bekannt. Es handelt sich hierbei um (1,2-Diphenyl-ethylendiamin)-platin(II)-Komplexverbindungen der allgemeinen Formel

...

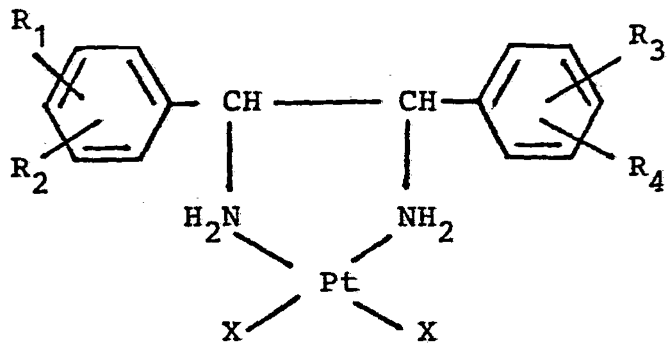

worin die Reste $R_1$, $R_2$, $R_3$ und $R_4$ gleich oder verschieden sind und Wasserstoff, Hydroxygruppen, $C_1$-$C_6$-Alkoxygruppen, gegebenenfalls durch Halogenatome oder $C_1$-$C_4$-Alkansulfonyloxygruppen substituierte $C_2$-$C_6$-Alkanoyloxygruppen oder $C_3$-$C_6$-Alkenoyloxygruppen bedeuten, wobei mindestens einer der Reste $R_1$, $R_2$, $R_3$ oder $R_4$ kein Wasserstoffatom ist, und X für das Äquivalent eines physiologisch verträglichen Anions steht.

Die Erfindung betrifft neue (1-Benzyl-ethylendiamin)-platin(II)-Komplexe, die als Arzneimittelwirkstoffe verwendbar sind.
Die neuen erfindungsgemäßen Verbindungen besitzen eine ausgeprägte Antitumorwirkung bei guter Verträglichkeit.
Die Wirkung zeigt sich insbesondere bei folgenden Tier- und Zellkulturmodellen:
Mäuseleukämie P 388, Mäuseleukämie L 1210, Cisplatinsensitives retikuläres Mäuse-Zellsarkom M 5076, hormonunabhängige, menschliche Brustkrebszellinie MDA-MB 231.

...

Die erfindungsgemäßen Verbindungen verhindern beziehungsweise hemmen sowohl das Wachstum von vorhandenen Tumorzellen als auch die Bildung neuer Tumorzellen; weiterhin zerstören sie vorhandene Tumorzellen beziehungsweise führen zu deren Rückbildung und verhindern beziehungsweise schwächen die Bildung von Metastasen.

Im Vergleich zu den bekannten Verbindungen sind die erfindungsgemäßen Verbindungen bei vergleichbarer oder überlegener Wirkung erheblich weniger toxisch.

...

Die folgenden Angaben betreffen bevorzugte Ausgestaltungen der Erfindung:

Die $C_1$-$C_6$-Alkylgruppen, die vorkommenden Alkoxygruppen und die $C_1$-$C_6$-Alkanoyloxygruppen können gerade oder verzweigt sein und bestehen vorzugsweise aus 1 bis 4 C-Atomen. Dasselbe gilt auch, wenn $C_1$-$C_6$-Alkylgruppen Bestandteil anderer funktioneller Gruppen sind (zum Beispiel im Falle von $C_1$-$C_6$-Alkansulfonyloxy oder $C_1$-$C_6$-Alkylaminocarbonyl usw.) Als Alkanoyloxygruppe kommt insbesondere die Acetoxygruppe in Frage. Als Halogensubstituenten kommen insbesondere Brom, Chlor und/oder Fluor in Frage. Bei der Trihalogenmethylgruppe handelt es sich vorzugsweise um Trifluormethyl. Vorzugsweise handelt es sich bei dem Thiophenrest um den Thienyl-(2)-rest, bei dem Indolrest um den Indolyl-(3)-rest und bei dem Imidazolrest um den Imidazolyl-(4)-rest. Falls einer oder mehrere der Reste $R_1$, $R_2$, $R_3$ oder $R_4$ eine Alkylgruppe bedeuten, handelt es sich vorzugsweise um eine Methylgruppe, eine Ethylgruppe oder eine Isopropylgruppe. Im Falle der Phenylethylgruppe handelt es sich vorzugsweise um die 1-Phenylethylgruppe. Bei der $C_1$-$C_6$-Alkyl-$C_1$-$C_6$-alkanoylaminogruppe handelt es sich um eine Aminogruppe, die am Stickstoff den $C_1$-$C_6$-Alkylrest und den $C_1$-$C_6$-Alkanoylrest enthält. Das Analoge gilt im Falle der $C_1$-$C_6$-Alkyl-$C_1$-$C_6$-alkansulfonylaminogruppe.

Besonders günstige Wirkung besitzen solche Verbindungen der Formel I, worin B ein Monochlorphenylrest, beispielsweise ein 4-Chlor-phenylrest ist.

Falls einer oder mehrere der Reste $R_5$, $R_6$ und $R_7$ eine Carboxygruppe oder eine Sulfogruppe enthalten, können die Verbindungen der Formel I auch in Form der ent-

...

sprechenden Salze mit physiologisch verträglichen Kationen vorliegen. Als solche Kationen kommen zum Beispiel in Frage: Kationen der Alkalimetalle (Na, K), der Erdalkalimetalle (Ca, Mg), Kationen von aliphatischen Mono-, Di-, Tri- oder quartären $C_1$-$C_6$-Alkylaminen, die auch durch Phenyl substituiert sein können, $NH_4^{\oplus}$.

Falls einer oder mehrere der Reste $R_5$, $R_6$ und $R_7$ eine Ammoniumgruppe oder die Gruppe $(C_1\text{-}C_6\text{-Alkyl})_3N^{\oplus}$ darstellen, liegen die Verbindungen der Formel I in Form der Salze mit physiologisch verträglichen Anionen vor. Hierbei handelt es sich beispielsweise um solche Anionen, die auch für die Gruppe X in Frage kommen.

Die Reste X stellen die bekannten und üblichen physiologisch verträglichen und pharmazeutisch verwendbaren Anionen ein- oder mehrwertiger Säuren dar. Insbesondere kommen beispielsweise die Anionen folgender Säuren in Frage: HBr, HCl, HJ, HF, $HNO_3$, $H_2SO_4$ ($SO_4^{--}$); $H_3PO_4$ ($HPO_4^{--}$); $H_2CO_3$, ($CO_3^{--}$); Kampfersulfonsäure, aliphatische oder aromatische Sulfonsäuren, beispielsweise $C_1$-$C_6$-Alkylsulfonsäuren (zum Beispiel Methansulfonsäure, Ethan-, Propan- oder Hexansulfonsäure), Benzol- oder Naphthalinsulfonsäure, die gegebenenfalls ein- oder zweifach durch Methylgruppen substituiert sind (Toluolsulfonsäure, insbesondere o- oder p-Toluolsulfonsäure); aliphatische $C_1$-$C_4$-Monocarbonsäuren, die gegebenenfalls ein-, zwei- oder dreifach durch Halogenatome (insbesondere Cl, F) substituiert sind (zum Beispiel Ameisensäure, Essigsäure, Propionsäure, Chloressigsäure, Dichloressigsäure, Trifluoressigsäure, Trichloressigsäure); aliphatische $C_2$-$C_{11}$-Dicarbonsäuren, die gegebenenfalls eine Doppelbindung

...

enthalten (zum Beispiel Oxalsäure, Malonsäure, 2-Amino-Malonsäure, Malonsäure, welche in 2-Stellung durch eine Benzylgruppe oder eine oder zwei $C_1$-$C_4$-Alkylgruppen substituiert ist, Maleinsäure, Fumarsäure, Bernsteinsäure); aliphatische Monohydroxy- und Dihydroxy-Monocarbonsäuren mit 2 bis 6, insbesondere 2 bis 3 Kohlenstoffatomen, wobei es sich vorzugsweise um $\alpha$-Monohydroxycarbonsäuren handelt wie Milchsäure, Glycerinsäure oder Glykolsäure; aliphatische Monohydroxy- und Dihydroxy-, Di- und Tricarbonsäuren mit 3 bis 8 Kohlenstoffatomen, insbesondere 3 bis 6 Kohlenstoffatomen wie Äpfelsäure, Weinsäure, Malonsäure, die an dem mittelständigen C-Atom durch eine Hydroxygruppe und gegebenenfalls eine $C_1$-$C_4$-Alkylgruppe substituiert sein kann, Isozitronensäure oder Zitronensäure; Phthalsäure, die gebenenfalls durch eine Carboxygruppe (insbesondere in 4-Stellung) substituiert ist; Gluconsäure; Glucuronsäure; die natürlichen $\alpha$-Aminosäuren (zum Beispiel L-Asparaginsäure); 1,1-Cyclobutandicarbonsäure; Organophosphorsäuren, wie Aldose- und Ketosephosphorsäuren (beispielsweise die entsprechenden Mono- und Diphosphorsäuren) zum Beispiel Aldose-6-phosphorsäuren wie D- oder L-Glucose-6-phosphorsäure, $\alpha$-D-Glucose-1-phosphorsäure, D-Fructose-6-phosphorsäure, D-Galactose-6-phosphor-säure, D-Ribose-5-phosphorsäure, D-Fructose-1,6-diphosphor-säuren; Glycerinphosphorsäuren (wobei der Phosphorsäurerest an einem der endständigen oder an dem mittelständigen Glycerinsauerstoffatom gebunden ist) wie $\alpha$-D,L-Glycerin-phosphorsäure, ß-Glycerinphosphorsäure; N-Phosphono-acetyl-Asparaginsäure.

...

Als Säuren für die Anionen X kommen weiterhin in Frage: Aromatische Carbonsäuren, die eine oder mehrere Carboxygruppen enthalten sowie außerdem noch $C_1$-$C_4$-Alkoxygruppen und/oder Hydroxygruppen. Falls sich mehrere Carboxygruppen an dem aromatischen Rest (zum Beispiel Benzolring) befinden, stehen mindestens 2 Carboxygruppen bevorzugt zueinander in Nachbarstellung. Falls der Benzolring zum Beispiel 4 oder 5 Carboxygruppen enthält, können Komplexe entstehen, die pro 1 Mol des Benzolcarbonsäureanions 2 Mol der Platin-Komponente enthalten. 2 benachbarte Carboxygruppen neutralisieren jeweils 1 Mol der Platinkomponente, so daß zum Beispiel im Falle der Benzolpentacarbonsäure die 1- und 2-ständigen sowie die 4- und 5-ständigen Carboxygruppen jeweils 1 Mol der Platinkomponente absättigen (zusammen also 2 Mol), während die freie 3-ständige Carboxygruppe frei oder in der Salzform mit einem physiologisch verträglichen Kation (zum Beispiel Alkalikation, insbesondere Natriumkation) vorliegt. Dies gilt ganz allgemein, wenn die Anionen X noch zusätzliche Säurefunktionen besitzen, die nicht für die Absättigung des Platins gebraucht werden. Das Analoge gilt für den Fall der Benzolhexacarbonsäure, wobei hier gegebenenfalls 1 Mol dieser Säure 3 Mol der Platinkomponente absättigen kann.

Beispiele für solche Säuren sind: Benzolmonocarbonsäure, Benzoldicarbonsäuren, Benzoltricarbonsäuren (zum Beispiel Trimellithsäure), Benzoltetracarbonsäuren, Benzolpentacarbonsäure, Benzolhexacarbonsäure; Syringasäure, Orotsäure.

...

Ebenfalls kommen als Säuren, die die Anionen X bilden, Aminosäuren beziehungsweise Aminosäurederivate, deren basische Aminogruppe durch eine Säuregruppe neutralisiert ist, in Frage. Es handelt sich hierbei zum Beispiel um Aminosäuren der folgenden Struktur:

$$R' - \underset{\displaystyle NH_2}{\underset{|}{CH}} - CO_2H$$

worin R' Wasserstoff, einen Phenylrest, einen Indolyl-(3)-methylrest, Imidazolyl-(4)-methylrest, eine $C_1$-$C_{10}$-Alkylgruppe oder eine $C_1$-$C_{10}$-Alkylgruppe, die durch eine Hydroxygruppe, eine Carboxygruppe, eine $C_1$-$C_6$-Alkoxygruppe, eine Mercaptogruppe, eine $C_1$-$C_6$-Alkylthiogruppe, eine Phenylgruppe, eine Hydroxyphenylgruppe, eine $C_2$-$C_6$-Alkanoylaminogruppe oder eine $C_1$-$C_6$-Alkoxycarbonylgruppe substituiert ist, bedeutet.

Die basische Aminogruppe in 2-Stellung ist hierbei durch eine übliche Aminosäureschutzgruppe neutralisiert (acyliert), beispielsweise durch einen $C_2$-$C_6$-Alkanoylrest oder den Butyloxycarbonylrest.
Falls in der obigen Formel R' eine Alkylgruppe ist, handelt es sich vorzugsweise um eine $C_1$-$C_6$-Alkylgruppe, die zum Beispiel in 2-, 3-, 4-, 5- oder 6-Stellung (Zählung beginnt an der Verknüpfungsstelle des Alkylrestes mit dem Restmolekül) eine $C_2$-$C_6$-Alkanoylaminogruppe, einen Imidazolyl-(4)-methylrest oder einen Indolyl-(3)-methylrest enthält. Einzelbeispiele für solche Aminosäuren sind: Leucin (vorzugsweise D- und L-Form), Valin (vorzugsweise D- und L-Form), Phenylalanin (vorzugsweise D- und L-Form), Phenylglycin (vorzugsweise D- und L-Form), Alanin (vorzugsweise D- und L-Form), Isoleucin (vorzugsweise D- und L-Form),

...

Asparagin (vorzugsweise D- und L-Form), Lysin (vorzugsweise D- und L-Form), Tryptophan (vorzugsweise D- und L-Form), Tyrosin (vorzugsweise D- und L-Form), Ornithin (vorzugsweise D- und L-Form).

Hierbei sind die basischen Aminogruppen durch eine übliche Acylaminoschutzgruppe blockiert, insbesondere durch die Acetylgruppe oder die Butyloxycarbonylgruppe.

Die Formel I umfaßt auch die möglichen Enantiomere und Diastereomere. Falls die Verbindungen Racemate sind, können diese in an sich bekannter Weise, zum Beispiel mittels einer optisch aktiven Säure in die optisch aktiven Isomere gespalten werden. Es ist aber auch möglich, von vornherein enantiomere oder gegebenenfalls auch diastereomere Ausgangsstoffe einzusetzen, wobei dann als Endprodukt eine entsprechende reine optisch aktive beziehungsweise diastereomere Verbindung erhalten wird. Unabhängig von der Struktur der Reste X besitzt bereits der 1-Benzyl-ethylendiamin-Teil 1 asymmetrisches Kohlenstoffatom und kann daher in der Racemat-Form oder in links- beziehungsweise rechtsdrehender Form vorliegen. Zusätzliche Formen können durch verschiedene enantiomere beziehungsweise diastereomere Formen der Reste X entstehen.
Hinsichtlich des Platinatoms handelt es sich bei den erfindungsgemäßen Verbindungen der Formel I stets um die cis-Verbindungen.

Das Ausgangsamin II wird beispielsweise als Racemat, als reine rechts- beziehungsweise linksdrehende Form oder in einer sonstigen diastereomeren Form eingesetzt.

...

Diese Konfiguration bleibt bei der Herstellung des Platinkomplexes erhalten.

Das Verfahren zur Herstellung der erfindungsgemäßen Verbindungen I wird in einem Lösungsmittel bei Temperaturen zwischen 0 und 90° C, vorzugsweise 10 bis 60° C, insbesondere 20 bis 50° C durchgeführt. Als Lösungsmittel kommen beispielsweise in Frage: Wasser, $C_1$-$C_6$-Alkanole (Methanol, Ethanol, tert.-Butanol), Tetrahydrofuran, Dioxan, Dimethylsulfoxid, Dimethylformamid, Ethylenglykoldimethylether, Diethylenglykoldimethylether sowie Gemische dieser Lösungsmittel, insbesondere Mischungen mit Wasser.

Die beiden Reaktionskomponenten (Platin-Verbindung und Verbindung II) werden vorzugsweise in äquimolaren Mengen eingesetzt. Der pH-Wert der Reaktionslösung soll zwischen 5 und 7, vorzugsweise bei pH 6 liegen. Die Einstellung des pH-Wertes erfolgt insbesondere durch Zusatz von Alkali, vorzugsweise wäßriger Natronlauge oder Kalilauge oder beispielsweise auch mittels Natriumcarbonat beziehungsweise durch Zusatz von Säuren, vorzugsweise wäßriger Salzsäure.

Als Tetrahalogen-platin(II)-Verbindungen (Säure sowie Komplexsalze) kommen die entsprechenden Tetrachloro-, Tetrabromo- und Tetrajodo-Verbindungen in Frage. Falls Platin(II)-halogenide als Ausgangskomponente eingesetzt werden, kommen die gleichen Halogenatome in Frage.

...

Als einwertige Kationen kommen in Betracht: Alkali-Ionen, insbesondere Natrium und Kalium; es können aber auch die Lithium, Rubidium, Cäsium verwendet werden, ebenso $NH_4^+$, $NR_4^+$, $PR_4^+$ oder $AsR_4^+$, in denen R ein $C_1$-$C_6$-Alkylrest oder ein Phenylrest ist. Zweiwertige Kationen können sein: Erdalkali-Ionen, insbesondere $Mg^{2+}$ und $Ca^{2+}$, aber auch $Zn^{2+}$. Als Platin(II)-halogenide kommen beispielsweise $PtCl_2$, $PtBr_2$ und $PtJ_2$ in Frage.

Die Verbindung II wird entweder in Form des Diamins oder in Form eines Säureadditionssalzes eingesetzt: zum Beispiel als Monohydrochlorid oder Dihydrochlorid, Mono- oder Dihydrobromid, Mono- oder Dihydrojodid oder als Salz mit einer anderen üblichen Säure. Insbesondere kommen auch die Säuren in Frage, deren Anionen die Reste X bilden. Weiterhin kann das Diamin in Form des Acetats beziehungsweise Diacetats eingesetzt werden, wobei gegebenenfalls vor dem Mischen der Reaktionskomponenten Kaliumchlorid (beispielsweise 2 Mol pro 1 Mol Verbindung II) zugesetzt wird. Ebenso kann das Diamin II in Form des Carbonats eingesetzt werden.

...

In Verbindungen der Formel I können vorhandene Hydroxygruppen und/oder Aminogruppen beziehungsweise $C_1$-$C_6$-Alkylaminogruppen des Restes B durch $C_1$-$C_6$-Alkanoylgruppen oder durch $C_1$-$C_6$-Alkansulfonylgruppen acyliert werden. In Hydroxygruppen des Restes B kann in gleicher Weise auch die Benzoylgruppe eingeführt werden. Diese Acylierung kann beispielsweise mittels Phosgen, $C_1$-$C_6$-Alkanoylhalogeniden, Benzoylhalogeniden, $C_1$-$C_6$-Alkansulfonylhalogeniden oder den Anhydriden der gesättigten aliphatischen $C_1$-$C_6$-Monocarbonsäuren bei Temperaturen zwischen 10 und 80° C, insbesondere 20 - 30° C in Anwesenheit üblicher säurebindender Stoffe erfolgen. Insbesondere kommen als säurebindende Stoffe aliphatische tertiäre Amine wie zum Beispiel Diisopropylethylamin in Betracht. Als inerte Lösungs- beziehungsweise Suspensionsmittel für die Acylierung kommen beispielsweise in Frage: niedere aliphatische Halogenkohlenwasserstoffe (Chloroform), aprotische Lösungsmittel wie Amide, $C_1$-$C_4$-Alkylamide und $C_1$-$C_4$-Dialkylamide aliphatischer $C_1$-$C_4$-Carbonsäuren (Dimethylformamid, Dimethylacetamid), N-Methyl-pyrrolidon, Dimethylsulfoxid oder Mischungen dieser Mittel.

Man kann diese Acylierung zum Beispiel aber auch in einem Zweiphasensystem beispielsweise Wasser/Chloroform durchführen, wobei der unter Zuhilfenahme eines Anionenaustauschers gewonnene acylierte Platin(II)-Komplex sich unlöslich abscheidet und das Gemisch von Säurechlorid und tertiärem Amin (Diisopropylethylamin) sich in der Chloroformphase befindet. Als Säurehalogenide kommen vorzugsweise die entsprechenden Chloride, Bromide und gegebenenfalls Jodide in Betracht. Als Anhydride kommen in Frage: Benzoesäureanhydrid sowie die Anhydride von $C_1$-$C_6$-Carbonsäuren, zum Beispiel symmetrische Säureanhydride wie Acetanhydrid, Propionsäureanhydrid, Buttersäureanhydrid.

...

Der Austausch der Liganden X gegen andere Liganden kann beispielsweise mittels der Silberhalogenidfällung erfolgen. Hierzu wird beispielsweise eine Dihalogeno-(1-benzyl-ethylendiamin)-platin(II)-Verbindung der Formel I, worin X Halogen (Chlor, Brom oder Jod) bedeutet in einem Lösungs- oder Suspensionsmittel bei Temperaturen zwischen 0 bis 90° C, vorzugsweise 10 bis 50° C, insbesondere 30 bis 40° C, vorzugsweise 40° C mit den Silbersalzen einer anderen Säure, die der Bedeutung X entspricht, umgesetzt. Man kann hierbei aber auch als Silbersalz Silbernitrat (zum Beispiel wäßrige Silbernitrat-Lösung) verwenden und erhält einen ionischen Diaquokomplex der Formel

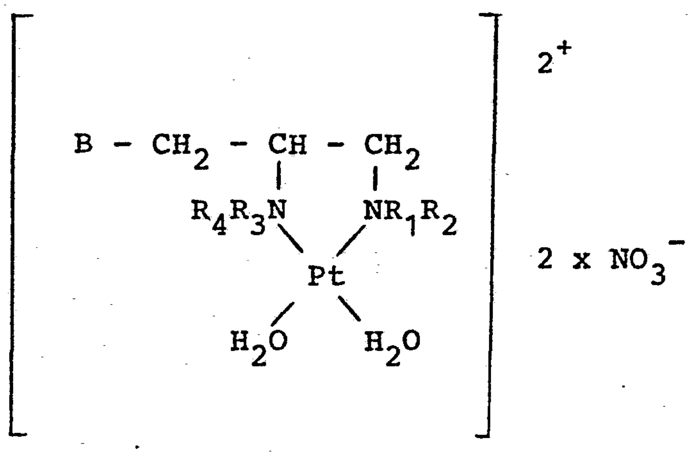

Aus diesem Komplex läßt sich der schwach gebundene Ligand Wasser leicht durch affinere Anionen (zum Beispiel $Cl^-$, $Br^-$ in Form von NaCl, KCl, NaBr, KBr, Malonat$^{2-}$, Chloracetat$^{(-)}$, Oxalat$^{2-}$, 1,1-Cyclobutandicarbonsäure-Anion$^{2-}$ sowie die übrigen angegebenen Säurereste X verdrängen, angewandt in Form der Säuren oder ihrer Salze, insbesondere ihrer Alkalisalze.

Die gleichen Verbindungen lassen sich auch durch Umsetzung äquimolarer Mengen von HX und Nitrat-freiem

...

Platinkomplex (letzterer unter Verwendung von Anionenaustauschern in der Hydroxidform, zum Beispiel Dowex 1 - 8X) gewinnen.

Ein Austausch der Abgangsgruppe (zum Beispiel $SO_4^{2-}$- beziehungsweise Oxalatanion$^{2-}$) ist im Falle der Sulfato- beziehungsweise Oxalato-(1-benzyl-ethylendiamin)-platin(II)-Verbindungen auch durch Umsetzung mit Erdalkalisalzen, die den gewünschten X-Liganden (zum Beispiel Glycerinsäure) enthalten, möglich, sofern der entstehende Komplex wasserlöslich ist und damit die Abtrennung des schwer wasserlöslichen Erdalkalisulfats oder -oxalats erlaubt.
Für dieses Verfahren geeignete X-Liganden sind vorzugsweise die Anionen von Hydroxycarbonsäuren, Sulfonsäuren, Halogenessigsäuren, Salpetersäure.

Die Lösungs- beziehungsweise Suspensionsmittel, die für das Herstellungsverfahren der Verbindungen I angegeben wurden, kommen auch für die Austauschreaktion in Frage (insbesondere eignen sich Wasser und Dimethylformamid sowie ferner Methanol, Ethanol, tert.-Butanol). Die Austauschreaktion wird beispielsweise in einem pH-Bereich zwischen 3 und 7 durchgeführt.

...

Herstellung von Ausgangssubstanzen der Formel II, worin B ein gegebenenfalls substituierter Phenylrest oder ein für B in Frage kommender heterocyclischer Rest ist.

Die Herstellung dieser Verbindungen (optisch aktive Verbindungen und Racemate) kann zum Beispiel analog der bekannten Herstellung von Benzylethylendiamin (D- und L-Form, Racemat) gemäß folgender stereospezifischer Synthese erfolgen:
Veresterung des im Phenylkern entsprechend substituierten Phenylalanins beziehungsweise des in ß-Stellung durch einen Thiophenrest, Indolrest oder Imidazolrest substituierten Alanins (D- oder L-Form oder Racemat) durch Rückflußkochen mit Thionylchlorid in Methanol. Die Methylester fallen als Hydrochlorid an (Helv. Chim. Acta 39, 1421 (1956)). Mehrmaliges Sättigen der methanolischen Lösung mit Ammoniak bei -5° C bis 0° C führt zu den entsprechenden Amiden (J. Amer. Chem. Soc. 53, 3183 (1931); J. Biol. Chem. 199, 801 (1952); Inorg. Chem. 18, 206 (1979)), wobei der Chlorwasserstoff der Ester-Hydrochloride teilweise am Amid gebunden bleibt. Unter den Reaktionsbedingungen fällt auch ein Teil als Ammoniumchlorid aus, das durch fraktionierte Kristallisation abgetrennt werden kann. Im dritten Reaktionsschritt werden die α-Aminosäureamide zu den Diaminen reduziert. Dies geschieht durch Zugabe der festen Amide zu einer Suspension von Lithiumaluminiumhydrid in Tetrahydrofuran, Rückflußkochen und anschließender Hydrolyse (Helv. Chim. Acta 38, 2036, 1955; Inorg. Chem. 18, 206, 1979; Gazz. Chim. Ital. 85, 1354, 1955; Bull. Chem. Soc. Jap. 49, 101, 1976) oder zu $Al(BH_4)_3$ in Dioxan (in situ hergestellt aus $NaBH_4$ und $AlCl_3$; siehe J. Am. Chem. Soc. 78, 2582, 1956). Die Hydrolyseprodukte des Lithiumaluminiumhydrids werden abfiltriert und die Diamine durch Abziehen des Lösungs-

...

mittels als zähe Öle isoliert. Sie lassen sich durch Destillation unter vermindertem Druck reinigen oder durch Umsetzung mit konzentrierter Salzsäure oder mit etherischer Salzsäure in die Hydrochloride überführen. Dieses Verfahren kann zum Beispiel analog der in der Dissertation von Manfred Schmidt, 1984, Universität Regensburg, Seite 119-22 beschriebenen Weise erfolgen.

Die für diesen Syntheseweg eingesetzten substituierten Phenylalanine können zum Beispiel auf folgende Weise erhalten werden:
Im ersten Schritt, einer "Azlacton-Synthese nach E. Erlenmeyer jun.", werden die durch die Reste $R_5$, $R_6$ und $R_7$ substituierten Benzaldehyde mit N-Acetyl- beziehungsweise N-Benzoylglycin in der Hitze unter Einwirkung von Natriumacetat in Essigsäureanhydrid zu den ungesättigten Azlactonen (Oxazolonen) kondensiert (Org. React. 3, 198 (1946)).
Die zweite Stufe, die Überführung der Azlactone in die entsprechenden α[N-Acylamino]zimtsäuren, erfolgt in zwei Schritten, und zwar über eine Verseifung mit verdünnter Natronlauge und anschließender Fällung mit verdünnter Salzsäure. Dabei ist es wichtig, sowohl stark saure (J. Amer. Chem. Soc. 64, 885 (1942)) beziehungsweise basische Bedingungen (J. Biol. Chem. 82, 438 (1929)) als auch langes Erhitzen (J. Biol. Chem. 82, 438 (1929)) zu vermeiden, da ansonsten die primär entstehenden α[N-Acylamino]zimtsäuren zu α-Ketosäuren hydrolysiert werden. Aufgrund der hohen Stabilität der ungesättigten Azlactone, besonders der 2-Phenylazlactone, lassen sich diese Bedingungen allerdings nicht ganz vermeiden, was sich gegebenenfalls in niedrigeren Ausbeuten widerspiegelt.

...

In der dritten Reaktionsstufe werden die Zimtsäuren zu den Benzoyl- beziehungsweise Acetyl-Aminosäuren reduziert. Dazu werden die Zimtsäuren in Wasser suspendiert und bei Raumtemperatur mit festem Natriumamalgam versetzt (Ber. Dtsch. Chem. Ges. 3638 (1899)). Die Acetyl- beziehungsweise Benzoylaminosäuren können daraufhin, nach Abdekantieren der entstandenen Lösung vom Quecksilber, mit Salzsäure ausgefällt werden.

Die Hydrolyse der N-Benzoylgruppe beziehungsweise der N-Acetylgruppe im letzten Reaktionsschritt wird durch mehrstündiges Kochen der Säuren in 20 % Salzsäure erreicht. Die substituierten Aminosäuren fallen dann nach Zugabe von Ammoniak bis zu einem schwach sauren pH-Bereich aus. Sie können durch Lösen in Säure und Ausfällen mit Ammoniak oder Natriumacetat umkristallisiert werden.
Dieses Verfahren kann zum Beispiel analog der in der Dissertation von Manfred Schmidt, 1984, Universität Regensburg, Seite 111-114 beschriebenen Weise erfolgen.

Ein anderer Weg ist der folgende:
In dieser auf zwei Stufen reduzierten Synthese werden die durch die Reste $R_5$, $R_6$ und $R_7$ substituierten Benzaldehyde mit Hydantoin (Molverhältnis 1:1) und geschmolzenem, wasserfreiem Natriumacetat bei 160° C innerhalb von 15 Stunden nahezu quantitativ zu den entsprechenden Benzalhydantoinen umgesetzt. Diese stabilen, meist gelblichen Feststoffe werden durch Reduktion und gleichzeitige Ringaufspaltung mit Ammoniumsulfid bei 100° C in einem Rührautoklaven innerhalb von 60 Stunden direkt in die $\alpha$-Aminosäuren überführt.

...

Dieses Verfahren kann zum Beispiel analog der in der Dissertation von Manfred Schmidt, 1984, Universität Regensburg, Seite 116-119 beschriebenen Weise erfolgen.

Die hierbei eingesetzten Ausgangsaldehyde können beispielweise aus den durch die Reste $R_5$, $R_6$ und $R_7$ substituierten Benzol-Derivaten durch folgende, bekannte Umsetzung erhalten werden: Durch Behandeln mit Paraformaldehyd in konzentrierter Salzsäure wird eine Chlormethylgruppe eingeführt, dann wird die Chlormethylgruppe mittels Natronlauge zur Hydroxymethylgruppe hydrolysiert (falls hierbei Mischungen von isomeren Hydroxymethyl-Verbindungen entstehen, können diese chromatographisch getrennt werden, zum Beispiel mit $CH_2Cl_2$/Ether 1:1), anschließend wird dann die Hydroxymethylgruppe mittels frisch gefälltem aktivem Braunstein zur Aldehydgruppe oxydiert. Dieses Verfahren kann zum Beispiel analog der in der Dissertation von Manfred Schmidt, 1984, Universität Regensburg, Seite 115-116 beschriebenen Weise erfolgen.

Benzylethylendiamine, die im Phenylkern freie Hydroxygruppen enthalten, werden zweckmäßig aus den entsprechenden Benzylethylendiaminen, bei denen einer oder mehrere der Reste $R_5$, $R_6$ und $R_7$ Methoxygruppen sind (die auf die zuvor angegebene Weise hergestellt werden können) durch Etherspaltung mit Bortribromid, zum Beispiel in Methylenchlorid-Suspension bei -60° C erhalten (E. von Angerer, Habilitationsschrift, Universität Regensburg 1983). Zweckmäßig werden die so erhaltenen Dihydrobromide der Hydroxyverbindungen nach Freisetzung der Amine in Methanol beziehungsweise Ethanol mit etherischer Salzsäure oder in Ether mit Chlorwasserstoffgas in die entsprechenden Dihydrochloride der Diamine II überführt, worin B die durch die Reste $R_5$, $R_6$ und $R_7$ substituierte Phenylgruppe ist und

...

in diesem Fall mindestens einer der Reste $R_5$, $R_6$, $R_7$ eine Hydroxygruppe darstellt.

Die Diamine II, in denen $R_1$ oder $R_1$ und $R_2$ $C_1$-$C_6$-Alkylgruppen, Benzylgruppen oder Phenylethylgruppen sind, werden aus den entsprechenden $\alpha$-Aminosäureamiden erhalten, bei deren Darstellung anstelle von Ammoniak ein $C_1$-$C_6$-Alkylamin, ein $C_1$-$C_6$-Dialkylamin, Benzylamin oder Phenylethylamin verwendet wird. Hierbei ist es gegebenenfalls vorteilhaft, die $\alpha$-Aminogruppe der eingesetzten $\alpha$-Aminosäure mit einer üblichen Schutzgruppe (zum Beispiel dem Benzyloxycarbonylrest) zu schützen. Die Umsetzung der so erhaltenen geschützten $\alpha$-Aminosäure mit dem Amin $HNR_1R_2$ erfolgt zweckmäßig in bekannter Weise in Gegenwart von N-Hydroxysuccinimid und Dicyclohexylcarbodiimid. Die $\alpha$-Amino-Schutzgruppe wird dann vor der Reduktion mit $LiAlH_4$ in bekannter Weise acidolytisch (zum Beispiel mit gesättigter Bromwasserstofflösung in Eisessig) abgespalten. Wird hingegen im Falle des Benzyloxycarbonylrestes (als Schutzgruppe für die $\alpha$-Aminogruppe) diese nicht vorher abgespalten, sondern direkt das geschützte Säureamid mit $LiAlH_4$ reduziert, erhält man entsprechende Amine der Formel II, worin der Rest $R_3$ eine Methylgruppe ist ($R_4$ = H, $R_1$, $R_2$ = H, $C_1$-$C_6$-Alkyl, Benzyl oder Phenylethyl). Amine der Formel II, worin $R_3$ und/oder $R_4$ $C_1$-$C_6$-Alkylgruppen sind ($R_1$ und $R_2$ haben die hierfür angegebenen Bedeutungen) können beispielsweise in bekannter Weise auch durch Mono- und Dialkylierung der entsprechenden Säureamide der Formel $B-CH_2-CH(NH_2)-CO-NR_1R_2$ und anschließende Reduktion mit $LiAlH_4$ erhalten werden. Zur Darstellung der Diamine II, in denen $R_3$ beziehungsweise $R_3$ und $R_4$ oder $R_1$ und $R_3$

...

Methylgruppen sind, werden die entsprechenden α-Aminosäureamide oder die entsprechenden Diamine zum Beispiel mit Chlorameisensäuremethylester (Bull. Chem. Soc. Jpn. 53, 2275 (1980)) beziehungsweise durch reduktive Alkylierung mit Formaldehyd in Gegenwart von Ameisensäure (Org. Synth. Coll. Vol. III, 723) mono- beziehungsweise dimethyliert, deren Reduktion mit Lithiumaluminiumhydrid in üblicher Weise (zum Beispiel nach der Dissertation von Manfred Schmidt, Universität Regensburg, 1984, Seite 121) die N-substituierten Diamine II ergibt. Bei dieser Reaktion wird gleichzeitig der Ameisensäuremethylesterrest in eine Methylgruppe überführt. Zur Einführung von vier Methylgruppen an den Stellen $R_1$, $R_2$, $R_3$ und $R_4$ werden die Diamine II mit $R_1$, $R_2$, $R_3$, $R_4$ = H mit Formaldehyd/Ameisensäure reduktiv alkyliert (Org. Synth. Coll. Vol. III, 723).

Diamine II, in denen $R_1$ und $R_3$ Isopropylgruppen sind, werden zum Beispiel synthetisiert, indem man sie in bekannter Weise mit Aceton in die Schiffbasen überführt und diese mit Lithiumaluminiumhydrid reduziert. Eine Isopropylgruppe an der Stelle $R_3$ kann auch eingeführt werden, indem man die Aminogruppe der α-Aminosäuren mit Aceton kondensiert und diese Derivate in der beschriebenen Weise mit Natriumborhydrid in die Diamine II (vergleiche Dissertation von Manfred Schmidt, 1984, Universität Regensburg, Seite 119-122) überführt. Dabei wird die Imin-Gruppe zur N-Isopropylgruppe reduziert. Entsprechend werden Benzyl- oder Phenylethylgruppen an der Stelle $R_3$ eingeführt, indem man die Aminogruppen der α-Aminosäurederivate mit Benzaldehyd oder Acetophenon kondensiert und anschließend mit $LiAlH_4$ reduziert.

Die Herstellung solcher Diamine der Formel II, worin B einen der angegebenen heterocyclischen Reste darstellt,

...

kann zum Beispiel aus den entsprechenden heterocyclischen Aldehyden (zum Beispiel Thienyl-(2)-aldehyd) oder den entsprechenden heterocyclischen Aminosäuren (das heißt Derivaten des Alanins, wobei das Alanin in ß-Stellung durch den entsprechenden heterocyclischen Rest substituiert ist) nach den vorstehend angegebenen Methoden erfolgen.

...

Durch Acylierung können in OH-Gruppen oder Aminogruppen, die sich an dem Phenylrest der Diamine II befinden, $C_1$-$C_6$-Alkanoyloxy-Gruppen eingeführt werden. (phenolische Hydroxygruppen können auch in Benzoyloxygruppen überführt werden) Acylierungsreagenzien sind dabei Säurehalogenide von aliphatischen $C_1$-$C_6$-Carbonsäuren, vorwiegend Säurechloride sowie Benzoylchlorid für den $C_6H_5COO$-Substituenten. Auch die entsprechenden Säureanhydride, zum Beispiel Acetanhydrid für die Bildung des $CH_3COO$-Substituenten, können verwendet werden. Entsprechend werden die $C_1$-$C_6$-Alkansulfonyl-Substituenten eingeführt, letztere zum Beispiel mit Hilfe der entsprechenden Sulfonsäurechloride. Bei diesen Acylierungen werden die beiden aliphatischen Aminofunktionen zweckmäßigerweise durch Iminbildung, zum Beispiel mit Benzaldehyd, geschützt. Nach der Acylierung kann die Imingruppe beispielsweise durch Hydrolyse mit verdünnter HCl in die Aminogruppe rückgespalten werden.

Zur Darstellung von Verbindungen, die in dem Rest B eine Carboxylfunktion oder eine Carbonylfunktion haben, wird von den entsprechenden Methylenoxy-alkyl-Verbindungen ausgegangen. Die Etherbindung im $CH_2OCH_3$-Substituenten bleibt während des gesamten Synthesewegs unverändert. Auf der Stufe des Diamins II wird die Etherspaltung zur $CH_2OH$-Verbindung, wie beschrieben, durchgeführt. Mit starken Oxidationsmitteln, zum Beispiel mit $CrO_3$, kann man die $CH_2OH$-Gruppe in die Carboxylgruppe überführen. Die Carboxylgruppe kann durch Neutralisation mit Basen in Carboxylatsalze physiologisch verträglicher Kationen umgewandelt werden, zum Beispiel mit Alkalihydroxyden, insbesondere NaOH, KOH oder Erdalkalihydroxyden, insbesondere $Mg(OH)_2$, $Ca(OH)_2$. Anstelle der Alkali- und Erdalkalihydroxyde können auch andere alkalisch reagierende Substanzen, zum Beispiel die Carbonate, verwendet werden. Auch das Ammoniumion $NH_4^+$ sowie substituierte Ammonium-

...

ionen $NR_4^+$ (R = Wasserstoff, $C_1$-$C_6$-Alkyl, Phenyl oder Benzyl) können eingeführt werden entweder durch Umsetzung der Carboxyl-Verbindungen mit den entsprechenden Aminen oder durch Ionenaustausch mit Ionenaustauschern in der $NR_4^+$-Form.

Mit Hilfe von $C_1$-$C_6$-Alkoholen läßt sich die Carboxylgruppe verestern und durch Umsetzung mit Ammoniak oder Aminen $NR_3$ (R = Wasserstoff, $C_1$-$C_6$-Alkyl) in die entsprechenden Säureamide überführen. Durch Einwirkung von wasserentziehenden Mitteln, zum Beispiel $P_2O_5$, entsteht aus der Carbonylaminogruppe die Cyanogruppe.

Bei der schonenden Oxidation der wie vorstehend beschrieben erhaltenen Verbindungen des Typs II mit $CH_2OH$-Gruppen, zum Beispiel nach Oppenauer mit Aceton/Aluminiumisopropoxid, bilden sich Formylderivate. Verwendet man bei der Darstellung den Ethersubstituenten CHR-O-$CH_3$ (R = $C_1$-$C_6$-Alkyl), so lassen sich die entsprechenden Verbindungen durch Etherspaltung und Oxidation der entstehenden alkoholischen Gruppe in die $C_1$-$C_6$-Carbonylsubstituierten Verbindungen umwandeln.

Amino-Substituenten $NR_2$ (R = Wasserstoff, $C_1$-$C_6$-Alkyl) werden eingeführt, indem man die Synthese von Diaminen II mit den entsprechenden Aminosubstituenten beginnt, die Substituenten aber, sofern sie noch NH-Bindungen enthalten, durch Benzoylierung, zum Beispiel mit Benzoylchlorid, schützt. Die Benzoyl-Gruppen am Aminosubstituenten werden zwar während der $LiAlH_4$-Reduktion zu Benzylgruppen reduziert. Diese können jedoch hydrogenolytisch, zum Beispiel unter Einwirkung von Wasserstoff mit Hilfe des Katalysators Platin auf Kohlenstoff, abgespalten werden.

...

Durch Alkylierung können die NH-Gruppen der Amino-Substituenten in NR-Gruppen (R = $C_1$-$C_6$-Alkyl) umgewandelt werden. Durch erschöpfende Alkylierung, zum Beispiel mit Dimethylsulfat, oder durch Ansäuern mit starken Säuren wie Salzsäure oder Schwefelsäure, lassen sich alle Amino-Substituenten $NR_2$ in die entsprechenden Ammonium-Substituenten $NR_3$ überführen, in denen R Wasserstoff, $C_1$-$C_6$-Alkyl sein kann. Aminogruppen $NH_2$ und NHR (R = $C_1$-$C_6$-Alkyl), die noch ein oder zwei H-Atome enthalten, können durch Acylierung, wie bei den OH-Verbindungen beschrieben, in die monoacylierten Substituenten NHCOR' und NRCOR' (R' = $C_1$-$C_6$-Alkyl) umgewandelt werden. Dies geschieht nach Herstellung der Komplexe I, in denen die koordinierten $NH_2$-Gruppen des Ethylendiamin-Molekülteils gegenüber Acylierungsmitteln eine stark reduzierte Reaktivität aufweisen. Statt Acylierungsmitteln können auch Sulfonylierungsmittel eingesetzt werden unter Bildung der $C_1$-$C_6$-Alkansulfonylamino-Gruppen.

Die Oxidation einer Aminogruppe am aromatischen Ring des Benzylsubstituenten B kann mit Hilfe der Methoden, angegeben in Houben-Weyl, Methoden der Organischen Chemie, Stickstoffverbindungen I, Teil I, Thieme-Verlag Stuttgart 1971, Seite 843-849 erfolgen, zum Beispiel mit Persäuren, wie Peroxidischwefelsäure (zum Beispiel Ammonium-peroxidisulfat), Peroximonoschwefelsäure, Peroxiessigsäure, Peroxitrifluoressigsäure, Peroximaleinsäure. Die Reaktion kann zum Beispiel in Schwefelsäure, Eisessig oder halogenierten Kohlenwasserstoffen ($CH_2Cl_2$) bei Temperaturen zwischen 10 - 150° C, vorzugsweise 10 - 80° C, insbesondere 10 - 20° C durchgeführt werden.

Die Sulfogruppe überdauert in Form des $SO_3^-$-Anions die zu den Diaminen II und den Platinkomplexen I führenden Reaktionsschritte. Sie kann durch Ansäuern mit starken

...

Säuren in die $SO_3H$-Form überführt werden. Zugabe von Basen oder alkalisch reagierender Stoffe ergibt die entsprechenden Sulfonate mit physiologisch verträglichen Kationen (analog den Carboxylaten). Veresterung der Sulfogruppe mit $C_1$-$C_6$-Alkoholen oder Amidierung mit Ammoniak, primären Aminen $RNH_2$ und sekundären Aminen $R_2NH$ ($R = C_1$-$C_6$-Alkyl) führt auf der Stufe der Diamine II zu Sulfonsäureestern und Sulfonsäureamiden mit den Substituenten $SO_3R$, $SO_2NH_2$, $SO_2NHR$, $SO_2NR_2$.

Die $C_1$-$C_6$-Alkansulfonyl-Substituenten, im Benzaldehyd zu Reaktionsbeginn vorhanden, werden bei den Reaktionen unter Bildung der Diamine II und der Platin-Komplexe I nicht angegriffen.

...

Zur weiteren Erläuterung wird auf die Herstellung einiger Ausgangsverbindungen der Formel II im Anschluß an die Beispiele 17 - 25 verwiesen sowie auf die Diplomarbeit von Ulrich Holzinger "Synthese und Untersuchung von N-Alkyl substituierten tumorhemmenden 1,2-Diamino-3-phenylpropan-dichloroplatin(II)-Komplexen", Fachbereich Chemie-Pharmazie der Universität Regensburg, 1985.
Die Herstellung anderer Ausgangsstoffe kann in analoger Weise erfolgen.

Die Ausgangsverbindung II, worin B der 4-Chlor-phenylrest und die Reste $R_1$, $R_2$, $R_3$ und $R_4$ Wasserstoff sind, kann beispielsweise wie folgt hergestellt werden (die Herstellung anderer entsprechender Ausgangsstoffe kann analog erfolgen):

4-Chlorphenylalaninmethylester-Hydrochlorid:
0,14 Mol (10 ml, 16,5 g) Thionylchlorid werden bei -5° C zu 100 ml Methanol getropft. Nach Zugabe von 0,11 Mol (22 g) 4-Chlorphenylalanin wird 15 Stunden unter Rückfluß erhitzt. Nach Abziehen des Lösungsmittels wird der weiße Rückstand am Hochvakuum getrocknet.
Die Umsetzung erfolgt quantitativ. Die Esterbildung kann IR-spektroskopisch am Auftreten einer Bande bei etwa 1750 $cm^{-1}$ verfolgt werden. Das Produkt wird ohne weitere Reinigung in die nächste Stufe eingesetzt.

4-Chlorphenylalaninamid:
0,1 Mol (25 g) 4-Chlorphenylalaninmethylester-Hydrochlorid werden in 250 ml Methanol gelöst. Dann wird bei 0° C Ammoniak mehrmals bis jeweils zur Sättigung

...

eingeleitet. Die Umsetzung wird nach 4 Tagen beendet. Das Lösungsmittel wird abgezogen und der weiße Rückstand getrocknet. Das Produkt wird in dieser Form in die nächste Stufe eingesetzt.

1,2-Diamino-3-(4-chlorphenyl)propan:

0,1 Mol (20 g) 4-Chlorphenylalaninamid beziehungsweise -Hydrochlorid werden in kleinen Portionen zu einer gut gerührten Suspension von 0,3 Mol $LiAlH_4$ in 250 ml trockenem Tetrahydrofuran gegeben. Die Mischung wird 24 Stunden unter Rückfluß gerührt und anschließend unter Eiskühlung tropfenweise mit 1,2 Mol $H_2O$ hydrolysiert. Nach einer weiteren Stunde Rühren bei Raumtemperatur wird von dem als schlammartigem Feststoff anfallenden Hydrolyseprodukt abfiltriert. Dieser Rückstand wird 24 Stunden mit 125 ml Tetrahydrofuran extrahiert. Filtrat und Extrakt werden vereinigt.

1,2-Diamino-3-(4-chlorphenyl)propan-Dihydrochlorid (Ausgangsstoff II):

Die Darstellung kann nach zwei unterschiedlichen Methoden erfolgen:

a) Die vereinigten Tetrahydrofuran-Lösungen (Filtrat und Extrakt) werden bis zur Hälfte eingeengt und anschließend tropfenweise unter Eiskühlung mit konzentrierter HCl versetzt. Das ausfallende Dihydrochlorid wird abfiltriert und getrocknet.

b) Die vereinigten Tetrahydrofuran-Lösungen werden eingeengt und der ölige Rückstand in 5-10 ml absolutem Ethanol gelöst. Daraufhin wird unter Eiskühlung und starkem Rühren mit 20 ml etherischer Salzsäure tropfenweise versetzt. Das Dihydrochlorid scheidet sich nach Zugabe von 20 ml Ether bei -40° C als feiner weißer Feststoff ab (F. 249 - 252° C, Zersetzung ab 240° C).

...

Die erfindungsgemäßen Verbindungen sind zur Herstellung pharmazeutischer Zusammensetzungen geeignet. Die pharmazeutischen Zusammensetzungen beziehungsweise Medikamente können eine oder mehrere der erfindungsgemäßen Verbindungen oder auch Mischungen derselben mit anderen pharmazeutisch wirksamen Stoffen enthalten. Zur Herstellung der pharmazeutischen Zubereitungen können die üblichen pharmazeutischen Träger- und Hilfsstoffe verwendet werden. Die Arzneimittel können zum Beispiel enteral, parenteral (zum Beispiel intravenös, intramuskulär, subkutan) oder oral angewendet werden. Beispielsweise kann die Verabreichung in Form von Tabletten, Kapseln, Pillen, Dragees oder Zäpfchen erfolgen. Als Liquida kommen zum Beispiel in Frage: Ölige oder wäßrige Lösungen oder Suspensionen (zum Beispiel in Sesam- oder Olivenöl), Emulsionen, injizierbare wäßrige und ölige Lösungen oder Suspensionen. Weiterhin können beispielsweise Trockenampullen, welche als Wirkstoff die erfindungsgemäße Verbindung I enthalten, hergestellt werden, wobei vor Gebrauch der Inhalt solcher Trockenampullen zum Beispiel in physiologischer Kochsalzlösung oder Gemischen aus physiologischer Kochsalzlösung und beispielsweise Dimethylsulfoxid aufgelöst wird.

...

Die erfindungsgemäßen Verbindungen zeigen beispielsweise am M 5076 Retikulumzellsarkom (Maus), an der P 388-Leukämie (Maus), an der L 5222 Leukämie (Ratte) und an der menschlichen MDA-MB 231-Brustkrebszellinie eine gute Antitumorwirkung. Beispielsweise wird mit der Verbindung gemäß Beispiel 4 beim Retikulumzellsarkom (Maus) bei subkutaner Verabreichung mit einer Dosis von 30,0 mg/kg Körpergewicht/Maus ein Rückgang der Ausgangstumorgröße erreicht. Bei der Testung an der hormonunabhängigen menschlichen Mammatumorzellinie MDA-MB 231 (siehe Dissertation von Manfred Schmidt, Universität Regensburg 1984, Seite 78, 168ff) zeigen die erfindungsgemäßen Verbindungen in vitro beispielsweise in Konzentrationen zwischen $1 - 5 \cdot 10^{-6}$ Mol/Liter, insbesondere $1{,}3 - 2{,}5 \cdot 10^{-6}$ Mol/Liter eine 50 %ige Wachstumshemmung an dieser Zellinie. In derselben Größenordnung liegt die Hemmung des $[^3H]$-Thymidineinbaus. Im Zellkulturversuch mit der Mäuseleukämie L 1210 zeigen die erfindungsgemäßen Verbindungen zum Beispiel in Dosierungen zwischen 0,01 - 0,2 µg/ml eine 50 %ige Wachstumshemmung.

Die Wirkungsrichtung der erfindungsgemäßen Verbindungen ist mit dem bekannten Mittel Cisplatin vergleichbar. Bei den erfindungsgemäßen Verbindungen sind beispielsweise jedoch die toxischen Nebenwirkungen erheblich geringer.

Die niedrigste, bereits wirksame Dosis am Retikulumzellsarkom der Maus ist beispielsweise $5 \cdot 3{,}2$ mg/kg intraperitoneal. Als allgemeiner Dosisbereich für die Wirkung (Retikulumzellsarkom/Maus) kommt beispielsweise in Frage: 3,2 - 30,0 mg/kg intraperitoneal beziehungsweise intravenös, insbesondere 15 mg/kg.

Indikationen, für die die erfindungsgemäßen Verbindungen

...

in Betracht kommen können: Tumorerkrankungen, besonders Mammacarcinom, Leukämie, Retikulumzellsarkom, ferner Ovarial-, Prostata- und Endometriumsarkom.

Die pharmazeutischen Zubereitungen enthalten im allgemeinen zwischen 100 bis 200, vorzugsweise 150 mg der erfindungsgemäßen aktiven Komponente.

Die Verabreichung kann beispielsweise in Form von Tabletten, Kapseln, Pillen, Dragees, Zäpfchen, Salben, Gelees, Cremes, Puder, Stäubepulver, Aerosolen oder in flüssiger Form erfolgen. Als flüssige Anwendungsformen kommen zum Beispiel in Frage: Ölige oder alkoholische beziehungsweise wässrige Lösungen sowie Suspensionen und Emulsionen. Bevorzugte Anwendungsformen sind Tabletten, die zwischen 100 und 200 mg oder Lösungen, die zwischen 0,02 bis 0,04 % an aktiver Substanz enthalten.

Die Einzeldosis der erfindungsgemäßen aktiven Komponenten kann beispielsweise liegen

a) bei oralen Arzneiformen zwischen 100 bis 200 mg, vorzugsweise 150 mg,

b) bei parenteralen Arzneiformen (zum Beispiel intravenös, intramuskulär) zwischen 100 bis 200 $mg/m^2$ Körperoberfläche, vorzugsweise 150 $mg/m^2$ Körperoberfläche,

c) bei Arzneiformen zur rektalen oder vaginalen Applikation zwischen 1 bis 5 %, vorzugsweise 2,5 %,

d) bei Arzneiformen zur lokalen Applikation auf die Haut und Schleimhäute (zum Beispiel in Form von

...

Lösungen, Lotionen, Emulsionen, Salben und so weiter) zwischen 1 bis 5 %, vorzugsweise 2,5 %.

- (Die Dosen sind jeweils bezogen auf die freie Base) -

Beispielsweise können 3 mal täglich 1 bis 4 Tabletten mit einem Gehalt von 100 bis 200 mg wirksamer Substanz oder zum Beispiel bei intravenöser Injektion 1000 ml mit einem Wirkstoffgehalt entsprechend 100 bis 200 $mg/m^2$ Körperoberfläche empfohlen werden. Bei oraler Verabreichung ist die minimale tägliche Dosis beispielsweise 300 mg; die maximale tägliche Dosis bei oraler Verabreichung soll nicht über 800 mg liegen.

Die akute Toxizität der erfindungsgemäßen Verbindungen an der Maus (ausgedrückt durch die LD 50 mg/kg; Methode nach Miller und Tainter; Proc. Soc. Exper. Biol. a. Med. 57 (1944) 261) liegt beispielsweise bei intraperitonealer Applikation bei 464 mg/kg.

Die Arzneimittel können in der Humanmedizin und in der Veterinärmedizin allein oder im Gemisch mit anderen pharmakologisch aktiven Stoffen verwendet werden.

Beispiele

Allgemeine Vorschrift zur Herstellung von cis-Diamin-dichloro-platin(II)-Komplexen der Formel

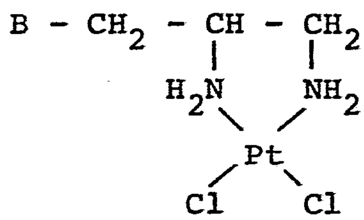

worin B die Bedeutungen gemäß Anspruch 1 hat.

Beispiele 1 bis 16 von Tabelle 1.

1 mMol (415 mg) Kaliumtetrachloroplatinat(II) wird in 5 ml Wasser gelöst. Der Reaktionskolben wird mit Alu-Folie gegen Lichteinfall geschützt. Unter Rühren wird bei Raumtemperatur eine Lösung von 1 mMol der Verbindung II (als Di- beziehungsweise Monohydrochlorid) in 5 ml Wasser zugetropft. Die Lösung wird auf etwa 50° C erwärmt, der pH-Wert mit 0,5 N NaOH auf etwa 6 eingestellt und mittels eines pH-Meters beziehungsweise pH-Indikatorpapier kontrolliert und gegebenenfalls durch Zugabe von 0,5 N NaOH bei 6 gehalten. Nach etwa 5 Stunden wird vom Niederschlag abfiltriert und mit viel $H_2O$ und Ethanol gewaschen. Die meist gelben Feststoffe werden am Hochvakuum bei 80° C getrocknet. Das häufig noch orange Filtrat wird noch 20 Stunden gerührt, wobei noch erhebliche Mengen Niederschlag ausfallen. Nach erneutem Abfiltrieren wird der Vorgang zum Teil ein weiteres Mal wiederholt. Die hergestellten Komplex-Verbindungen sind in der Tabelle 1 aufgeführt. Die Schmelzpunkte aller

...

0193083

in der Tabelle 1 aufgeführten Platinkomplexe liegen über 250° C.

Falls in Tabelle 1 nichts anderes angegeben ist, handelt es sich bei den Komplexen jeweils umd die DL-Formen (Racemate).

Die Komplexe, die in der Tabelle 1 aufgeführt sind, besitzen eine hellgelbe Farbe.

Tabelle 1

| Beispiel-Nr. | Gruppe $-CH_2-B$ | Charakteristische IR-Schwingungsbanden in $cm^{-1}$ (KBr) | | | |
|---|---|---|---|---|---|
| | | $\nu$ N-H | $\delta$ N-H | $\nu$ Pt-N | $\nu$ Pt-Cl |
| 1 (D-Form) | $-CH_2C_6H_5$ | 3275 s, 3190 s, 3120 m | 1567 s | 587 m | 302 m |
| 2 (L-Form) | $-CH_2C_6H_5$ | 3275 s, 3190 s, 3120 m | 1567 s | 587 m | 302 m |
| 3 | $-CH_2C_6H_5$ | 3280 vs, 3200 s, 3110 w | 1565 s | 555 w | 305 m, sh, 300 m |
| 4 | 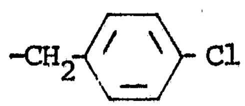 | 3265 vs, 3195 s, 3110 m | 1565 s | 568 | 310 m,sh, 300 m |

$\nu$ = Valenzschwingung
$\delta$ = Deformationsschwingung

s = stark, vs = sehr stark, m = mittelstark, sh = Schulter, br = breit,
w = schwach

...

Tabelle 1 (Fortsetzung)

| Beispiel-Nr. | Gruppe $-CH_2-B$ | Charakteristische IR-Schwingungsbanden in $cm^{-1}$ (KBr) $\nu$ N-H | $\delta$ N-H | $\nu$ Pt-N | $\nu$ Pt-Cl |
|---|---|---|---|---|---|
| 5 | 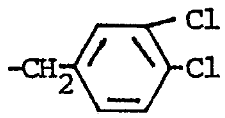 | 3270 vs, 3195 vs, 3110 m | 1565 s | 570 | 325 m, sh, 300 m, br |
| 6 | 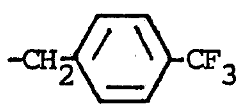 | 3265 s, 3195 s, 3110 m | 1565 s | 560 | 300 m, br |
| 7 | 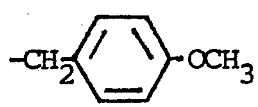 | 3270 vs, 3200 s, 3120 m | 1565 m | 580 m | 300 m,br |
| 8 | 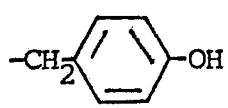 | 3265 vs, 3200 s, 3110 m,sh | 1565 s | 580 | 305 m,br |

Tabelle 1 (Fortsetzung)

| Beispiel-Nr. | Gruppe $-CH_2-B$ | Charakteristische IR-Schwingungsbanden in $cm^{-1}$ (KBr) ν N-H | δ N-H | ν Pt-N | ν Pt-Cl |
|---|---|---|---|---|---|
| 9 | 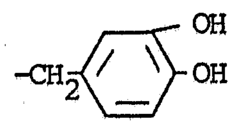 | 3280 vs, 3220 vs, 3140 s | 1575 s | | |
| 10 | 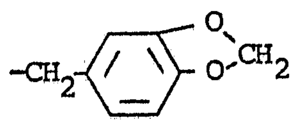 | 3260 s, 3190 s, 3105 s | 1560 m,br | 520 w | 320 w,sh, 300 w |
| 11 | 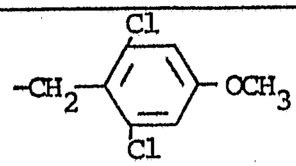 | 3280 vs, 3200 vs, 3120 vs, br | 1570 vs | | |
| 12 | 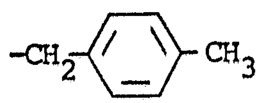 | 3275 s, 3195 s, 3115 w | 1567 s | 580 w | 300 m |

...

Tabelle 1 (Fortsetzung)

| Beispiel-Nr. | Gruppe $-CH_2-B$ | Charakteristische IR-Schwingungsbanden in $cm^{-1}$ (KBr) | | | |
|---|---|---|---|---|---|
| | | $\nu$ N-H | $\delta$ N-H | $\nu$ Pt-N | $\nu$ Pt-Cl |
| 13 | 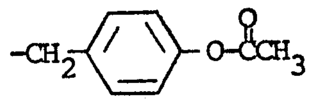 | 3265 vs, 3195 s, 3110 w | 1565 s | 570 w | 310 m |
| 14 | 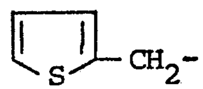 | 3270 vs, 3220 vs, 3110 m | 1570 s | | 325 m, 300 m |
| 15 | 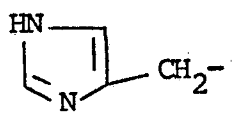 | 3200 vs,br, 3130 vs,br | 1585s,br | 615 m | 325 m,sh, 310 m,br |
| 16 (L-Form) | 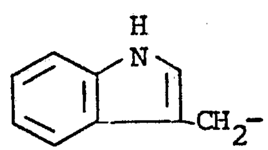 | 3410 s,br, 3350 s,sh,br, 3260 vs, 3190 vs, 3110 | 1585 s, 1575 s, 1565 s | | |

Beispiele 17 - 25

Allgemeine Herstellungsvorschrift:

Es werden hiernach die in Tabelle 2 aufgeführten Platin(II)-Komplexe der folgenden Formel hergestellt:

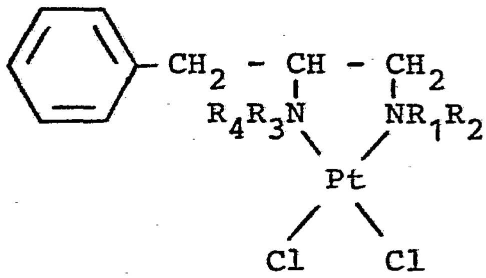

Die Komplexe werden als hellgelbe, feinkristalline Pulver erhalten, die sich sehr leicht elektrostatisch aufladen. Sie sind beispielsweise in $H_2O$, Ether, Essigester und ähnlichen Mitteln unlöslich, während die geringe Löslichkeit in $CHCl_3$, $CH_2Cl_2$, Aceton, Methanol und Ethanol mit der Anzahl der Alkylsubstituenten am Stickstoff zunimmt.

Gut löslich sind die Komplexe in Dimethylformamid und Dimethylsulfoxid.

Die Komplexe der Beispiele 17, 20, 21, 23, 24, 25 und 26 werden wie folgt hergestellt:

1 mMol (415 mg) Kaliumtetrachloroplatinat(II) werden in 5 ml Wasser gelöst. Unter Rühren wird bei Raumtemperatur tropfenweise eine neutralisierte Lösung (pH 6 - 7) von 1 mMol Ligand (Diamin oder Diamin-Dihydrochlorid) in 5 - 10 ml Wasser zugegeben. Die Lösung wird auf etwa 50° C erwärmt und der pH-Wert wird laufend kontrolliert. Falls nötig wird er mit 1 n HCl beziehungsweise 1 n NaOH auf pH = 6 nachgestellt.

...

Nach 10 - 30 Minuten beginnt ein hellgelber Niederschlag auszufallen. Nach 4 - 5stündigem Rühren wird der bis dahin gebildete Feststoff abfiltriert, mit viel Wasser und wenig (0,5 ml) Ethanol gewaschen. Die gelben Feststoffe werden im Hochvakuum bei 80° C getrocknet. Das noch orange gefärbte Filtrat wird weiter bei Raumtemperatur gerührt, wobei oft nur noch geringe Mengen an weiterem Produkt anfallen. Die Komplexe gemäß den Beispielen 17, 20, 23, 24 und 25 schmelzen oberhalb 250° C. Der Platin-Komplex gemäß Beispiel 21 zersetzt sich bei 190° C; der Platin-Komplex gemäß Beispiel 26 schmilzt bei 175° C.

Die Komplexe der Beispiele 18, 19, 22, 27 und 28 werden wie folgt hergestellt:
1 mMol (415 mg) $K_2PtCl_4$ werden in 5 ml Wasser gelöst und unter Rühren zu einer Lösung von 1 mMol Ligand-Diamin in 6 ml Dimethylformamid getropft. Sollte etwas Ligand ausfallen, wird mehr Dimethylformamid zugegeben (ca. 5 - 10 ml). Nach Zugabe von 1 ml Dimethylsulfoxid verändert sich die Farbe innerhalb von 10 Minuten von orange nach gelb. Bei Raumtemperatur engt man im Hochvakuum ein und versetzt den öligen Rückstand mit ca. 50 ml Wasser. Die gelben Komlexe fallen sofort aus und werden nach längerem Rühren bei Raumtemperatur (ca. 1 Tag) kristallin. Die Feststoffe werden abgesaugt, mit viel Wasser und wenig Ethanol gewaschen und im Hochvakuum bei 80° C getrocknet. Die Komplexe gemäß den Beispielen 18 und 19 schmelzen oberhalb 250° C. Der Komplex gemäß Beispiel 22 zersetzt sich bei 210° C. Die Komplexe gemäß Beispiel 27 und 28 schmelzen bei 125 beziehungsweise 110° C.

...

Tabelle 2

| Bei-spiel Nr. | $R_3$ | $R_4$ | $R_1$ | $R_2$ | Charakteristische IR-Schwingungsbanden in $cm^{-1}$ (KBr) $\nu$N-H | $\nu$C-H | $\delta$N-H | $\nu$C=C | $\delta$C-H | $\nu$Pt-N | $\nu$Pt-Cl |
|---|---|---|---|---|---|---|---|---|---|---|---|
| 17 | H | H | $CH_3$ | H | 3290 m<br>3180 s<br>3130 vs | 3070 m<br>3040 m 2940 w | 1580 m | 1610 s<br>1500 s | 750 s<br>700 s | 580 w<br>465 w | 310 m |
| 18 | $CH_3$ | H | H | H | 3280 w<br>3200 m<br>3120 vs | 3040 w 2960 w | 1580 m | 1605 w<br>1500 m | 745 s<br>730 s<br>695 s | 580 w<br>545 w<br>450 w | 310 m |
| 19 | $CH_3$ | H | $CH_3$ | H | 3120 vs | 3040 w 2950 w<br>2870 vw | 1585 vw | 1605 w<br>1500 m | 745 s<br>700 s | 565 w<br>450 vw | 310 m |
| 20 | H | H | $CH_3$ | $CH_3$ | 3210 vs<br>3180 vs<br>3120 s | 3020 m 2940 m<br>2870 vw | 1585 m | 1605 w<br>1500 s | 755 s<br>705 s | 585 m<br>545 m<br>460 m | 310 s |
| 21 | $CH_3$ | $CH_3$ | H | H | 3120 vs | 3030 m 2940 m<br>2860 w | 1585 w | 1605 m<br>1500 s | 740 s<br>700 s | 580 m<br>450 m | 310 s |

...

Fortsetzung Tabelle 2

| Bei-spiel Nr. | $R_3$ | $R_4$ | $R_1$ | $R_2$ | Charakteristische IR-Schwingungsbanden in $cm^{-1}$ (KBr) ν N-H | ν C-H | | δ N-H | ν C=C | δ C-H | ν Pt-N | ν Pt-Cl |
|---|---|---|---|---|---|---|---|---|---|---|---|---|
| 22 | $CH_3$ | $CH_3$ | $CH_3$ | $CH_3$ | fehlt | 3030 w<br>3000 m | 2940 s<br>2850 m | fehlt | 1605 w<br>1500 m | 740 vs<br>700 s | 580 w<br>435 w | 315 s |
| 23 | H | H | $CH(CH_3)_2$ | H | 3180 s<br>3120 vs | 3040 w | 2980 s | 1585 w | 1605 w<br>1500 m | 750 s<br>700 s | 580 s<br>465 s | 315 s |
| 24 | $CH(CH_3)_2$ | H | H | H | 3280 m<br>3200 vs | 3040 w | 2980 w | 1580 s | 1605 w<br>1500 m | 750 s<br>700 s | 585 w<br>460 w | 315 s |
| 25 | $CH(CH_3)_2$ | H | $CH(CH_3)_2$ | H | 3275 m<br>3190 s<br>3130 vs | 3040 s | 2980 vs | 1580 s | 1610 w<br>1500 s | 750 s<br>700 vs | 580 m<br>460 m | 315 s |
| 26 | $CH_3$ | H | $CH(CH_3)(C_6H_5)$ | H | 3150 s | 3060 m<br>3030 m | 2980 w<br>2930 w | 1580 w | 1605 w<br>1500 s | 750 s<br>740 s<br>700 vs | 585 vw<br>520 w | 310 m |
| 27 | $CH(CH_3)(C_6H_5)$ | H | H | H | 3230 s | 3080 m<br>3040 s | 2990 m<br>2930 w | 1590 w | 1610 w<br>1500 m | 750 s<br>700 vs | 595 vw<br>440 m | 330 m |

Fortsetzung Tabelle 2

| Bei-spiel Nr. | $R_3$ | $R_4$ | $R_1$ | $R_2$ | Charakteristische IR-Schwingungsbanden in $cm^{-1}$ (KBr) | | | | | | | |
|---|---|---|---|---|---|---|---|---|---|---|---|---|
| | | | | | $\nu$N-H | | $\nu$C-H | $\delta$N-H | $\nu$C=C | $\delta$C-H | $\nu$Pt-N | $\nu$Pt-Cl |
| 28 | $CH_2C_6H_5$ | H | H | H | 3230 s<br>3190 s | 3040 s<br>3070 s | 2920 m | 1590 w | 1610 m<br>1500 m | 750 s<br>700 vs | 575 vw<br>440 m | 320 m |

...

Herstellung von Ausgangsaminen für die Beispiele 17-28

a) (S)-2-Amino-1-N-methylamino-3-phenylpropan

Unter Eiskühlung werden in kleinen Portionen 10 mmol (1,78 g) (L)-Phenylalaninmethylamid zu einer gerührten Suspension von 30 mmol (1,14 g) $LiAlH_4$ in ca. 50 ml trockenem Tetrahydrofuran gegeben. Die Mischung wird 24 Stunden am Rückfluß erhitzt. Um überschüssiges $LiAlH_4$ zu hydrolysieren gibt man nach dem Abkühlen auf $0^\circ$ C tropfenweise 120 mmol (2,16 ml) Wasser hinzu. Man filtriert über eine Nutsche ab und engt das Filtrat ein. Den Rückstand extrahiert man über Nacht mit 150 ml Tetrahydrofuran in einer Soxhlet - Apparatur. Man vereinigt das Extrakt mit dem Filtrat und destilliert im Vakuum das Lösungsmittel ab. Das leicht gelbliche Rohprodukt wird im Hochvakuum ($10^{-4}$ Torr) bei $75^\circ$ C Luftbadtemperatur destilliert. F. des Dihydrochlorids $89^\circ$ C.

Das hier eingesetzte (L)-Phenylalaninmethylamid wird wie folgt erhalten:

50 mmol (8,96 g) (L)-Phenylalaninmethylester werden in 75 ml absolutem Methanol gelöst und im Eis - Kochsalzbad auf $-10^\circ$ C abgekühlt. Dann wird die Lösung mit trockenem Methylamin (Siedepunkt $-7^\circ$ C) gesättigt und über Nacht gerührt. Das Abkühlen und Einleiten wiederholt man zweimal. Das Lösungsmittel wird im Vakuum abdestilliert, und das erhaltene ölige Produkt wird mit einem Essigester - Ether - Gemisch (1:1) umkristallisiert. Beim Stehenlassen bei $-20^\circ$ C kristallisiert ein weißer Feststoff aus. F. $57^\circ$ C.

b) (S)-2-Amino-1-N,N-dimethylamino-3-phenylpropan

Die Darstellung aus 12,5 mmol (3,41 g) (L)-Phenylalanindimethylamid-Hydrobromid (Herstellung ist weiter unten angegeben) und 37,5 mmol (1,42 g) $LiAlH_4$ wird analog a) durchgeführt. Hochvakuumdestillation bei $100^\circ$ C/$10^{-4}$ Torr (Luftbadtemperatur).

...

Die erhaltene Verbindung ist ein farbloses Öl.

N-Benzyloxycarbonyl-(L)-phenylalanin

100 mmol (16,51 g) (L)-Phenylalanin werden in 50 ml 2n NaOH gelöst, im Eisbad gekühlt und tropfenweise mit 106 mmol (15,04 ml) Chlorameisensäurebenzylester versetzt. Man rührt 30 Minuten bei Raumtemperatur. Fällt bereits Produkt aus, wird soviel Wasser hinzugegeben, bis eine klare Lösung erreicht ist. Man tropft 4n Salzsäure bis zur sauren Reaktion zu (pH = 4) und schüttelt dreimal mit Essigester aus. Die organischen Phasen werden über Natriumsulfat getrocknet und im Vakuum vom Lösungsmittel befreit. Man erhält ein farbloses, öliges Produkt. Es wird in 200 ml Chloroform gelöst, durch Zugabe von 400 ml Petrolether in der Kälte ausgefällt und in einer großen Nutsche abgesaugt. Farblose Kristalle.

Benzyloxycarbonyl-(L)-phenylalanindimethylamid

20 mmol (5,99 g) N-Benzyloxycarbonyl-(L)-phenylalanin und 24 mmol (2,76 g) N-Hydroxysuccinimid werden in 30 ml Chloroform gelöst und unter Eiskühlung und Rühren mit 22 mmol (4,34 g) Dicyclohexylcarbodiimid, gelöst in ca. 10 ml Chloroform, versetzt. Nach 10 Minuten Rühren wird in einem Eis-Kochsalzbad auf -10° C abgekühlt und 20 mmol (0,90 g) kondensiertes, trockenes Dimethylamin (Siedepunkt +7° C) zugegeben. Man rührt 24 Stunden, wobei man das Kühlbad langsam auftauen läßt. Der gebildete Niederschlag wird abfiltriert und das Filtrat nacheinander mit je 20 ml 2n Salzsäure, gesättigter $NaHCO_3$-Lösung und Wasser gewaschen. Nach dem Trocknen über Natriumsulfat und Abziehen des Lösungsmittels im Vakuum erhält man ein öliges Produkt. Es wird bei 240° C Luftbadtemperatur im Hochvakuum ($10^{-4}$ Torr) destilliert. Farbloses, sehr zähflüssiges Öl.

...

(L)-Phenylalanindimethylamid - Hydrobromid

13 mmol (4,28 g) Benzyloxycarbonyl-(L)-phenylalanindimethylamid werden mit 10 ml eiskalter gesättigter HBr - Eisessig - Lösung (40 %ig) übergossen und 60 Minuten bei Raumtemperatur gerührt. Die Reaktionsmischung wird im Vakuum eingeengt. Das farblose, ölige Produkt wird mit ca. 150 ml Ether versetzt und 15 Stunden bei -20° C stehengelassen. Es bildet sich ein weißer kristalliner Feststoff. Er wird abgesaugt, getrocknet und ohne weitere Reinigung zur anschließenden Reduktion verwendet.

c) (S)-2-Amino-1-N-isopropylamino-3-phenyl-propan

Die Darstellung aus 20 mmol (5,74 g) (L)-Phenylalaninisopropylamid - Hydrobromid (Herstellung siehe weiter unten) und 60 mmol (2,28 g) $LiAlH_4$ wird analog a) durchgeführt. Hochvakuumdestillation bei 145° C/ $10^{-4}$ Torr (Luftbadtemperatur). Das Amin ist ein farbloses Öl.

N-Benzyloxycarbonyl-(L)-phenylalaninisopropylamid

20 mmol (5,99 g) N-Benzyloxycarbonyl-(L)-phenylalanin und 24 mmol (2,76 g) N-Hydroxysuccinimid werden in 30 ml Chloroform gelöst und unter Eiskühlung und Rühren mit 22 mmol (4,54 g) Dicyclohexylcarbodiimid, gelöst in ca. 10 ml Chloroform, versetzt. Nach 10 Minuten Rühren bei Raumtemperatur werden 20 mmol (1,71 ml) Isopropylamin zugegeben. Nach 24 Stunden wird der gebildete Niederschlag abfiltriert und das Filtrat nacheinander mit 20 ml verdünnter Salzsäure, gesättigter $NaHCO_3$-Lösung und Wasser gewaschen. Der nach dem Trocknen über Natriumsulfat und Abdestillieren des Lösungsmittels erhaltene wachsartige Rückstand wird mit Essigester/Petrolether umkristallisiert. Farblose, kristalline Substanz.

...

(L)-Phenylalanin-isopropylamid - Hydrobromid

20 mmol (6,81 g) N-Benzyloxycarbonyl-(L)-phenylalanin-isopropylamid werden mit 15 ml eiskalter gesättigter HBr-Lösung (ca. 40 %ig) übergossen und 60 Minuten bei Raumtemperatur gerührt. Die Reaktionsmischung wird im Vakuum eingeengt. Das farblose, ölige Produkt wird mit ca. 150 ml Ether versetzt und 15 Stunden bei -20° C stehengelassen. Es bildet sich ein sehr hygroskopischer, weißer, kristalliner Feststoff. Er wird über eine Nutsche abgesaugt, getrocknet und ohne weitere Reinigung zur anschließenden Reduktion verwendet (farblose Kristalle).

d) (S)-2-N-Methylamino-1-(N-(S)-1-phenylethylamino)-3-phenylpropan

Die Darstellung aus 18,2 mmol (7,30 g) N-Benzyloxycarbonyl-(L)-phenylalanin-(S)-1-phenylethylamid (Herstellung siehe weiter unten) und 164 mmol (6,22 g) $LiAlH_4$ wird analog a) durchgeführt. Hydrolysiert wird unter Eiskühlung mit 656 mmol (11,80 ml) Wasser. Im Hochvakuum destilliert zuerst der entstandene Benzylalkohol ab (ca. 100° C). Das Diamin siedet bei ca. 150° C/$10^{-4}$ Torr (Luftbadtemperatur). Farbloses Öl.

N-Benzyloxycarbonyl-(L)-phenylalanin-(S)-1-phenylethylamid

Die Darstellung aus 20 mmol (5,99 g) N-Benzyloxycarbonyl-(L)-phenylalanin, 24 mmol (2,76 g) N-Hydroxysuccinimid, 22 mmol (4,54 g) Dicyclohexylcarbodiimid und 20 mmol (2,53 g) (S)-1-Phenylethylamin erfolgt analog c). Farbloser Feststoff, F. 99° C.

...

e) 1-Amino-(S)-2-N-methylamino-3-phenylpropan

Zu einer eisgekühlten Suspension von 90 mmol (3,41 g) $LiAlH_4$ in 100 ml Tetrahydrofuran werden in kleinen Portionen 10 mmol (2,36 g) N-Ethyloxycarbonyl-(L)-phenylalaninamid (Herstellung aus (L)-Phenylalaninamid und Chlorameisensäureethylester in Gegenwart von $Na_2CO_3$ im Eisbad) gegeben. Es wird 24 Stunden am Rückfluß erhitzt. Hydrolysiert wird unter Eiskühlung mit 360 mmol (6,50 ml) Wasser. Weitere Aufarbeitung analog a). Es wird bei 75° C Luftbadtemperatur im Hochvakuum ($10^{-4}$ Torr) destilliert. Farblose Flüssigkeit.

F. des Hydrochlorids 160° C.

f) 1-Amino-(S)-2-N,N-dimethylamino-3-phenylpropan

In 100 mmol (3,85 ml) 98 %iger Ameisensäure werden 20 mmol (3,28 g) (L)-Phenylalaninamid unter Rühren gelöst und mit 60 mmol (4,47 ml) 37 %iger wäßriger Formaldehyd-Lösung versetzt. Es wird kurz erwärmt, bis Kohlendioxidentwicklung einsetzt (ca. 2 bis 3 Minuten), und dann ohne Wärmezufuhr weitergerührt. Klingt die Gasentwicklung ab, wird noch 15 Minuten am Rückfluß auf 100° C erhitzt. Nach dem Abkühlen werden 20 ml 2n Salzsäure zugegeben und am Rotationsverdampfer von überschüssigen Edukten und Wasser befreit. Der gelbe, ölige

...

Rückstand wird in 7 ml Wasser aufgenommen und das Amin durch Addition von 5 ml 18n Natronlauge freigesetzt. Die organische Phase wird abgetrennt und die wäßrige Phase zweimal mit 10 ml Essigester ausgeschüttelt. Die kombinierten organischen Phasen werden über Kaliumcarbonat getrocknet und vom Lösungsmittel befreit. Man erhält 17,3 mmol (3,32 g) N,N-Dimethylamino-(L)-phenylalaninamid als gelb gefärbtes Öl. Dieses wird portionsweise zu einer eisgekühlten Suspension von 69 mmol (2,62 g) $LiAlH_4$ in ca. 100 ml Tetrahydrofuran gegeben und 24 Stunden am Rückfluß erhitzt. Hydrolysiert wird unter Eiskühlung mit 276 mmol (4,97 ml) Wasser. Weitere Aufarbeitung analog a). Das Produkt wird im Hochvakuum ($10^{-4}$ Torr) bei $90^{o}$ C Luftbadtemperatur destilliert (farbloses Öl).

g) <u>1-Amino-(S)-2-N-isopropylamino-3-phenylpropan</u>

20 mmol (3,20 g) (L)-Phenylalaninamid werden in 125 ml Benzol gelöst, mit 20 mmol (1,47 ml) Aceton und einer Spatelspitze p-Toluolsulfonsäure versetzt, und in einer Soxhlet - Apparatur, die mit $CaSO_4 \cdot 1/2\ H_2O$ als Trockenmittel gefüllt ist, 12 Stunden am Rückfluß erhitzt. Nach Abziehen des Lösungsmittels im Vakuum erhält man ein luftempfindliches zähes Öl als Kondensationsprodukt. Dieses wird unter Eiskühlung zu einer Suspension von 100 mmol (3,79 g) $LiAlH_4$ in 100 ml Tetrahydrofuran gegeben und über Nacht am Rückfluß erhitzt. Man hydrolysiert das überschüssige $LiAlH_4$ durch langsames Zutropfen von 400 mmol (7,20 ml) Wasser (im Eisbad). Man filtriert über eine Nutsche ab und engt das Filtrat ein. Den Rückstand extrahiert man über Nacht mit 150 ml Tetrahydrofuran in einer Soxhlet - Apparatur. Das Extrakt wird mit dem Filtrat vereinigt und das Lösungsmittel wird im Vakuum abdestilliert. Das erhaltene gelbliche Öl wird im Hoch-

...

vakuum bei 80° C Luftbadtemperatur destilliert (farblose Flüssigkeit).
Werden in der vorhergehenden Vorschrift anstelle von 20 mmol (1,47 ml) Aceton 20 mmol (2,33 ml) Acetophenon beziehungsweise 20 mmol (2,02 ml) Benzaldehyd eingesetzt, so entstehen die Verbindungen 1-Amino-(S)-2-(N-(R,S)-1-phenylethylamino)-3-phenylpropan (Destillation bei 150° C Luftbadtemperatur im Hochvakuum; farblose Flüssigkeit) beziehungsweise 1-Amino-(S)-2-N-benzylamino-3-phenyl-propan (Destillation bei 145° C Luftbadtemperatur im Hochvakuum; farblose Flüssigkeit).

h) <u>(S)-1,2-Bis(N-methylamino)-3-phenylpropan</u>

Es werden 225 mmol (9,00 g) NaOH in 45 ml Wasser gelöst, im Eisbad abgekühlt und mit 49 mmol (7,35 g) (S)-1,2-Diamino-3-phenylpropan, gelöst in ca. 45 ml Benzol, versetzt. Unter Rühren tropft man innerhalb von 30 Minuten eine Lösung von 108 mmol (10,3 ml) Chlorameisensäureethylester in 45 ml Benzol zu, und läßt bei Raumtemperatur 3 Stunden rühren. Die organische Phase wird abgetrennt, und die wäßrige Lösung zweimal mit 20 ml Benzol ausgeschüttelt. Die vereinigten Phasen werden über Natriumsulfat getrocknet und vom Lösungsmittel befreit. Man erhält 40,33 mmol (11,87 g) farblosen, kristallinen Feststoff /(S)-1,2-Bis(N-ethyloxycarbonylamino)-3-phenylpropan/. Dieser wird in kleinen Portionen zu einer eisgekühlten Suspension von 370 mmol (14,0 g) $LiAlH_4$ in 300 ml Tetrahydrofuran gegeben. Nach 24 Stunden Erhitzen unter Rückfluß wird bei Eiskühlung mit 1,5 Mol (27 ml) Wasser vorsichtig hydrolysiert. Die Hydrolyseprodukte filtriert man ab und engt das Filtrat ein. Den Rückstand extrahiert man 12 Stunden mit 150 ml Tetrahydrofuran in einer Soxhlet-Apparatur. Man vereinigt das

...

Extrakt mit dem Filtrat und destilliert im Vakuum das Lösungsmittel ab. Die leicht bräunliche Flüssigkeit wird im Hochvakuum bei 85° C destilliert. Farblose Flüssigkeit.

i) <u>(S)-1,2-Bis(N-isopropylamino)-3-phenylpropan</u>

21,2 mmol (3,18 g) (S)-1,2-Diamino-3-phenylpropan werden in 125 ml Benzol gelöst, mit 42,4 mmol (3,11 ml) Aceton und einer Spatelspitze p-Toluolsulfonsäure versetzt. In einer Soxhlet-Apparatur, die mit gebranntem Calciumsulfat als Trockenmittel gefüllt ist, wird 12 Stunden unter Rückfluß erhitzt. Nach Abziehen des Lösungsmittels im Vakuum erhält man ein leicht gelbliches Öl. Es wird in 50 ml Methanol gelöst, im Eis-Kochsalzbad auf -10° C abgekühlt und mit 80,0 mmol (3,03 g) $NaBH_4$ versetzt. Unter Rühren läßt man auf Raumtemperatur kommen und erhitzt noch 12 Stunden unter Rückfluß. Nach dem Abkühlen destilliert man das Lösungsmittel im Vakuum ab und hydrolysiert mit 30 ml Wasser. Es wird mit 150 ml Ether ausgeschüttelt und die organische Phase noch zweimal mit je 30 ml Wasser gewaschen. Nach dem Trocknen über Natriumsulfat zieht man den Ether ab und destilliert das erhaltene bräunliche Öl im Hochvakuum bei 100° C Luftbadtemperatur. Farbloses Öl.

<u>Darstellung der Diamin-Dihydrochloride</u>

Zu Reinigungszwecken werden die Diamine in ihre Dihydrochloride überführt. Die besten Ergebnisse wurden bei Einleiten von trockenem Chlorwasserstoff-Gas in eine gutgekühlte etherische Lösung des Diamins erhalten:
ca. 2 g Diamin werden in 5 ml Ethanol gelöst, mit 50 ml Ether versetzt und im Eis-Kochsalzbad auf -15° C gekühlt. Dann wird ein langsamer Strom von trockenem HCl-Gas bis

...

zum Ausscheiden eines weißen, kristallinen Niederschlags eingeleitet. Bei zu großem HCl-Überschuß fällt der Niederschlag ölig an. Das meist sehr feuchtigkeitsempfindliche Dihydrochlorid wird abgesaugt und im Hochvakuum getrocknet.

Überführung der Dihydrochloride in die Diamine

Ca. 10 mmol Dihydrochlorid werden mit 20 ml Ether und 10 ml Triethylamin 30 Minuten unter Rückfluß gekocht. Das ausgefallene Triethylammoniumchlorid wird abgesaugt und das Filtrat vom überschüssigen Lösungsmittel und Triethylamin im Vakuum befreit. Die erhaltenen Öle werden im Hochvakuum destilliert.

...

Die folgende Tabelle 3 mit den Beispielen 29 bis 36 betrifft nochmals Platinkomplexe der Formel

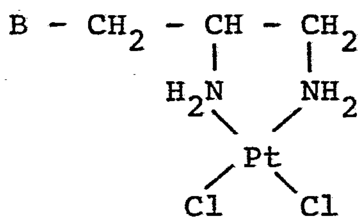

Die Herstellung dieser Verbindungen erfolgt nach der Vorschrift, die für die Beispiele 1 bis 16 angegeben ist.

...

Tabelle 3

| Beispiel-Nr. | Gruppe $-CH_2-B$ | Charakteristische IR-Schwingungsbanden in $cm^{-1}$ (KBr) | | | |
|---|---|---|---|---|---|
| | | $\nu$ N-H | $\delta$ N-H | $\nu$ Pt-N | $\nu$ Pt-Cl |
| 29 | 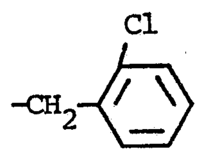 | 3280 $cm^{-1}$, vs<br>3200 $cm^{-1}$, s<br>3120 $cm^{-1}$, m | 1560 $cm^{-1}$, s | 570 $cm^{-1}$, w | 305 $cm^{-1}$, m, br |
| 30 | 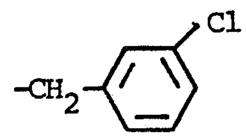 | 3270 $cm^{-1}$, vs<br>3190 $cm^{-1}$, s<br>3120 $cm^{-1}$, m | 1560 $cm^{-1}$, s, b | 560 $cm^{-1}$, w | 300 $cm^{-1}$, m, br |
| 31 | 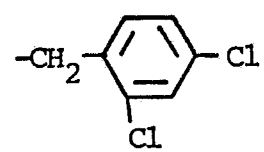 | 3270 $cm^{-1}$, s<br>3200 $cm^{-1}$, s<br>3120 $cm^{-1}$, m | 1560 $cm^{-1}$, s | 570 $cm^{-1}$, w | 300 $cm^{-1}$, m, br |
| 32 | 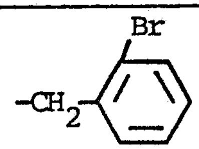 | 2260 $cm^{-1}$, s<br>2190 $cm^{-1}$, vs<br>2120 $cm^{-1}$, s | 1570 $cm^{-1}$, s | 590 $cm^{-1}$, m<br>440 $cm^{-1}$, m | 310 $cm^{-1}$, m, br |

Fortsetzung Tabelle 3

| Beispiel-Nr. | Gruppe $-CH_2-B$ | Charakteristische IR-Schwingungsbanden in $cm^{-1}$ (KBr) $\nu$ N-H | $\delta$ N-H | $\nu$ Pt-N | $\nu$ Pt-Cl |
|---|---|---|---|---|---|
| 33 | 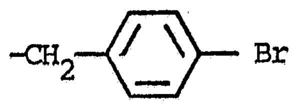 | 3270 $cm^{-1}$, s<br>3200 $cm^{-1}$, s<br>3120 $cm^{-1}$, m | 1560 $cm^{-1}$ | 590 $cm^{-1}$, s<br>470 $cm^{-1}$, m | 310 $cm^{-1}$, m, br |
| 34 | 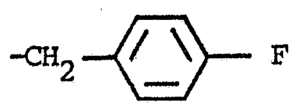 | 3270 $cm^{-1}$, vs<br>3190 $cm^{-1}$, s<br>3120 $cm^{-1}$, m | 1565 $cm^{-1}$ | 580 $cm^{-1}$, m<br>490 $cm^{-1}$, m | 300 $cm^{-1}$, m, br |
| 35 | 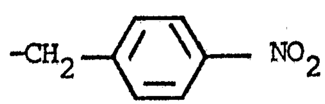 | 3260 $cm^{-1}$, s<br>3190 $cm^{-1}$, vs<br>3110 $cm^{-1}$, m | 1560 $cm^{-1}$, s | 590 $cm^{-1}$, w | 310 $cm^{-1}$, m, br |
| 36 | 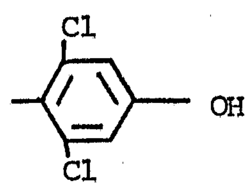 | 3270 $cm^{-1}$, s<br>3200 $cm^{-1}$, vs<br>3120 $cm^{-1}$, s | 1580 $cm^{-1}$, s | 560 $cm^{-1}$, m | 310 $cm^{-1}$, m |

Beispiel 37

Austausch des Anions X durch andere Anionen

1 mMol (416,2 mg) des Dichloro-Komplexes (DL-Form) der Formel

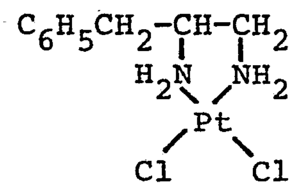

wird in 5 bis 10 ml $H_2O$ suspendiert und mit 2 mMol (339,7 mg) $AgNO_3$, gelöst in 5 ml $H_2O$, versetzt. Die Reaktionsmischung wird im Dunkeln gerührt. Bereits nach wenigen Stunden läßt sich im allmählichen Übergang der anfangs gelben Farbe des Dichloro-Komplexes zur weiß-grauen Farbe des sich bildenden Silberchlorids ein Fortschreiten der Umsetzung erkennen. Nach etwa zwei bis sieben Tagen wird das Silberchlorid abfiltriert, der pH-Wert durch Zugabe von 0,5 molarer Ammoniaklösung auf 4 bis 5 gebracht und das klare Filtrat mit 1 mMol der Säuren 1,1-Cyclobutandicarbonsäure, Malonsäure, Hydroxymalonsäure, Benzolsulfonsäure, Weinsäure, $\alpha$-Chloressigsäure, Asparaginsäure, Phthalsäure sowie 4-Carboxyphthalsäure beziehungsweise der Salze Natriumoxalat und Natriumisocitrat in fester Form versetzt. In den meisten Fällen beginnt bereits nach wenigen Stunden die Abscheidung der gelben oder weißen Komplexe, die noch 20 Stunden gerührt werden. Dann wird abfiltriert, mit eiskaltem Wasser gewaschen und am Hochvakuum bei 80° C getrocknet.
Auf diese Weise werden zum Beispiel Komplexe der obenangegebenen Formel erhalten, bei denen die beiden Chlor-Anionen durch die zweiwertigen Anionen der folgenden

...

Säuren ersetzt sind: Oxalsäure (weißes Pulver, F. $>$ 250° C); 1,1-Cyclobutandicarbonsäure (weißes Pulver, F. 250° C); Malonsäure (weißes Pulver, F. $>$ 250° C); Hydroxymalonsäure (weißes Pulver, F. $>$ 250° C); 4-Carboxy-phthalsäure (weißes Pulver, F. 222° C, Zersetzung); Phthalsäure (weißes Pulver, F. 185° C, Zersetzung); Isocitronensäure (hellgelbes Pulver, F. 226° C, Zersetzung); Weinsäure (weißes Pulver, F. 215° C, Zersetzung); Asparaginsäure (weißes Pulver, F. 130° C, Zersetzung).

Wegen der guten Wasserlöslichkeit des Dibenzolsulfonat-Komplexes, des $\alpha$-Chloracetat-nitrat-Komplexes und des Dinitrat-Komplexes wurde in diesen Fällen am Hochvakuum bis zur Bildung eines zähen Rückstandes eingeengt, der mit Ether versetzt wurde. Nach kurzem Rühren fielen diese Komplexe ebenfalls als weiße Feststoffe an, die abfiltriert und am Hochvakuum getrocknet wurden.

Der Benzolsulfonat-Komplex ($X = C_6H_5SO_3$) ist ein weißes Pulver, das bei 170° C unter Zersetzung schmilzt.
Der $\alpha$-Chloracetat-Nitrat-Komplex (Ein $X = NO_3$, das andere $X = CH_2Cl\text{-}CO_2$) ist ein beiges Pulver, das bei 148° C unter Zersetzung schmilzt.
Der Dinitrat-Komplex (mit 2 Mol $H_2O$) ist ein gelbes Pulver, F. 250° C.

...

Aus dem Dichloro-Komplex (DL-Form) der Formel

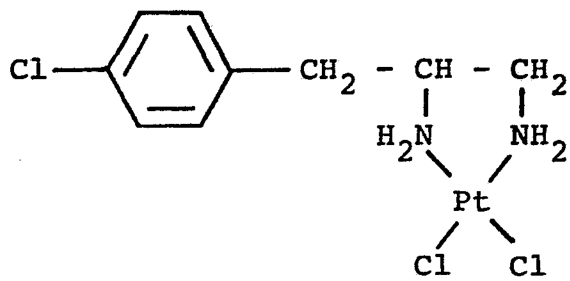

werden in analoger Weise zum Beispiel Komplexe erhalten, wo die zwei Anionen X die folgende Bedeutung haben:
Die beiden Anionen X bilden zusammen das Malonsäureanion ($^{\ominus}O_2C-CH_2-CO_2^{\ominus}$).

Der Komplex ist ein farbloser Feststoff;
Zersetzungspunkt 307° C.

Die beiden Anionen X bilden zusammen das Hydroxymalonsäureanion ($^{\ominus}O_2C-CH(OH)-CO_2^{\ominus}$).

Der Komplex ist ein farbloser, in Wasser löslicher Feststoff;
Zersetzungspunkt 270° C.

...

Komplex mit dem Tetra-Anion der 1,2,3,4,5-Benzolpentacarbonsäure (es handelt sich um einen Bis-Komplex, wobei 1 Mol der anionischen Verbindung mit 2 Mol der Platin-Komponente verbunden ist):

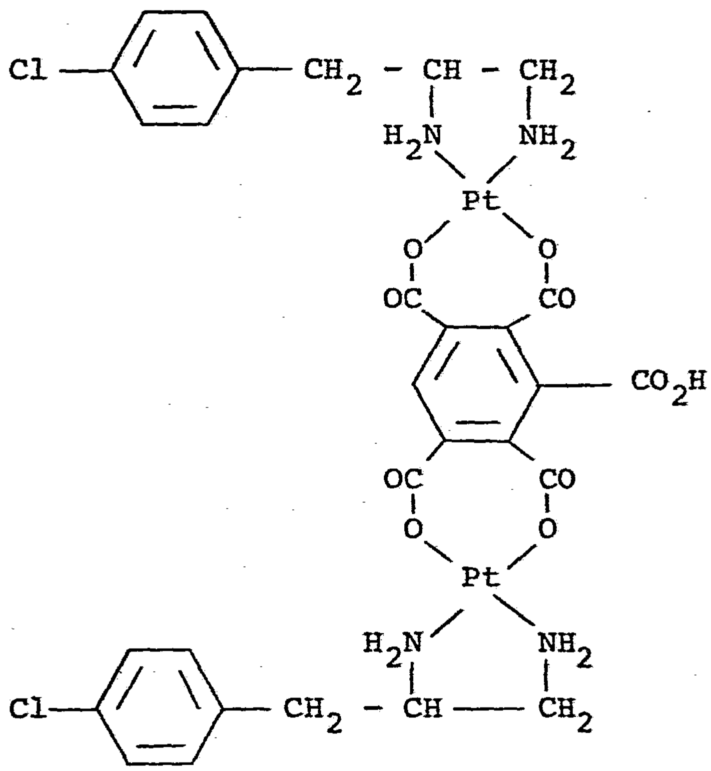

Der Komplex ist ein weißes Pulver, das oberhalb 160° C unter Zersetzung schmilzt.

Jedes Anion X ist das Anion der Aminosäure Ornithin (L-Form, deren beide Aminogruppen acetyliert sind. Der Komplex ist ein weißes Pulver; Schmelzpunkt 115° C (unter Zersetzung).

## Beispiele für galenische Zubereitungen

Überzogene Tabletten:

200 g Verbindung entsprechend Beispiel 3, 300 g Lactose D 10, 130 g Maisstärke und 10 g Magnesiumstearat werden durch ein Sieb mit einer Maschenweite von 0,8 mm gegeben und homogenisiert.
Diese Masse wird in bekannter Weise zu gewölbten Tabletten von 100 mg Gewicht gepreßt.
Zur Herstellung überzogener Tabletten werden diese Kerne mit Hilfe einer Sprüheinrichtung in bekannter Weise mit einem magen- beziehungsweise dünndarmlöslichen Überzug versehen, der aus einem geeigneten polymeren Filmbildner wie zum Beispiel Estern von Acrylaten oder Methacrylaten und geeigneten Hilfsstoffen wie Netzmitteln, Weichmachern, Farbstoffen, Gleitmitteln usw. bestehen kann. Die Kerne können auch in üblicher Weise zu Dragees verarbeitet werden. Eine Filmtablette beziehungsweise ein Dragee enthalten 20 mg Wirkstoff.

Lyophilisat:

In 800 ml Wasser für Injektionszwecke werden unter Rühren 50 g Mannit und 5 g (D,L)-Dibenzolsulfonato-(1-Benzylethylendiamin)-Platin II (Verbindung nach Beispiel 37) gelöst und mit Wasser für Injektionszwecke das Volumen auf 1 Liter aufgefüllt.
Diese Lösung wird unter aseptischen Bedingungen über ein Membranfilter von 0,22 µm Porenweite sterilfiltriert und zu 10 ml in 15 ml Injektionsflaschen der hydrolytischen Klasse I abgefüllt. Die Flaschen werden mit einem Gefriertrocknungsstopfen versehen und in einer geeigneten Anlage lyophilisiert. Nach der Trocknung begast man mit sterilem,

...

getrocknetem Stickstoff und verschließt die Flaschen in der Anlage. Die Stopfen werden durch eine Bördelkappe gesichert.
Für die intravenöse Anwendung wird das Lyophilisat in 10 ml Injektionswasser rekonstituiert.
1 ml Lösung enthält 5 mg Wirkstoff.

...

0193083

Degussa Aktiengesellschaft
Weissfrauenstraße 9, 6000 Frankfurt/Main

# Tumorhemmende (1-Benzyl-ethylendiamin)-platin(II)-Komplexe

Patentansprüche:

1. (1-Benzyl-ethylendiamin)-platin(II)-Komplexe der allgemeinen Formel

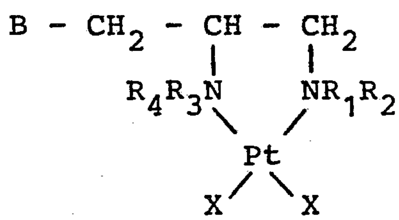

I

worin die Reste $R_1$, $R_2$, $R_3$ und $R_4$ gleich oder verschieden sind und Wasserstoff, eine $C_1$-$C_6$-Alkylgruppe, eine Benzylgruppe oder eine Phenylethylgruppe bedeuten, und B ein Thienylrest, ein Indolylrest, ein Imidazolylrest oder ein durch die Reste $R_5$, $R_6$ und $R_7$ substituierter Phenylrest ist und die Reste $R_5$, $R_6$ und $R_7$ gleich oder verschieden sind und Wasserstoff, Halogen, Trihalogenmethyl, $C_1$-$C_6$-Alkyl, Hydroxy, $C_1$-$C_6$-Alkoxy, Phenoxy, Benzyloxy, $C_1$-$C_6$-Alkanoyloxy, Benzoyloxy, $C_1$-$C_6$-Alkansulfonyloxy, Carboxy, $C_1$-$C_6$-Carbalkoxy, Cyano,

...

Aminocarbonyl, Aminocarbonyl, welches einen oder zwei $C_1$-$C_6$-Alkylreste enthält, $C_1$-$C_6$-Alkylcarbonyl, Nitro, Amino, $C_1$-$C_6$-Alkylamino, Di-$C_1$-$C_6$-Alkylamino, $(C_1$-$C_6$-Alkyl$)_3N^{\oplus}$, $C_1$-$C_6$-Alkanoylamino, $C_1$-$C_6$-Alkyl-$C_1$-$C_6$-alkanoylamino, $C_1$-$C_6$-Alkansulfonylamino, $C_1$-$C_6$-Alkyl-$C_1$-$C_6$-alkansulfonylamino, Aminosulfonyl, Aminosulfonyl, welches einen oder zwei $C_1$-$C_6$-Alkylreste enthält, $C_1$-$C_6$-Alkoxysulfonyl ($-SO_2-O-C_1-C_6$-Alkyl), Sulfo ($-SO_3H$) oder $C_1$-$C_6$-Alkansulfonyl bedeuten und zwei dieser Reste auch die Methylendioxygruppe sein können
und X für das Äquivalent eines physiologisch verträglichen Anions steht,
sowie gegebenenfalls deren Salze mit physiologisch verträglichen Kationen oder Anionen.

2. Verfahren zur Herstellung von (1-Benzyl-ethylendiamin)-platin(II)-Komplexen der allgemeinen Formel

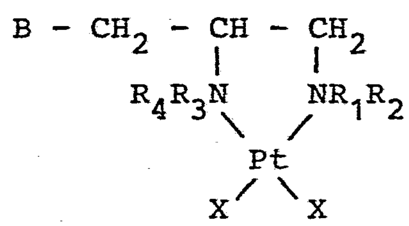

I

worin die Reste $R_1$, $R_2$, $R_3$ und $R_4$ gleich oder verschieden sind und Wasserstoff, eine $C_1$-$C_6$-Alkylgruppe, eine Benzylgruppe oder eine Phenylethylgruppe bedeuten, und B ein Thienylrest, ein Indolylrest, ein Imidazolylrest oder ein durch die Reste $R_5$, $R_6$ und $R_7$ substituierter Phenylrest ist und die Reste $R_5$, $R_6$ und $R_7$ gleich oder verschieden

...

sind und Wasserstoff, Halogen, Trihalogenmethyl, $C_1$-$C_6$-Alkyl, Hydroxy, $C_1$-$C_6$-Alkoxy, Phenoxy, Benzyloxy, $C_1$-$C_6$-Alkanoyloxy, Benzoyloxy, $C_1$-$C_6$-Alkansulfonyloxy, Carboxy, $C_1$-$C_6$-Carbalkoxy, Cyano, Aminocarbonyl, Aminocarbonyl, welches einen oder zwei $C_1$-$C_6$-Alkylreste enthält, $C_1$-$C_6$-Alkylcarbonyl, Nitro, Amino, $C_1$-$C_6$-Alkylamino, Di-$C_1$-$C_6$-alkylamino, $(C_1$-$C_6$-$Alkyl)_3N^{\oplus}$, $C_1$-$C_6$-Alkanoylamino, $C_1$-$C_6$-Alkyl-$C_1$-$C_6$-alkanoylamino, $C_1$-$C_6$-Alkansulfonylamino, $C_1$-$C_6$-Alkyl-$C_1$-$C_6$-alkansulfonylamino, Aminosulfonyl, Aminosulfonyl, welches einen oder zwei $C_1$-$C_6$-Alkylreste enthält, $C_1$-$C_6$-Alkoxysulfonyl ($-SO_2-O-C_1-C_6$-Alkyl), Sulfo ($-SO_3H$) oder $C_1$-$C_6$-Alkansulfonyl bedeuten und zwei dieser Reste auch die Methylendioxygruppe sein können

und X für das Äquivalent eines physiologisch verträglichen Anions steht,

sowie gegebenenfalls deren Salzen,

dadurch gekennzeichnet,

daß man eine Tetrahalogeno-platin(II)-säure, ein Tetrahalogeno-platin(II)-Komplexsalz mit zwei einwertigen oder einem zweiwertigen Kation oder ein Platin(II)-halogenid mit einer Verbindung der Formel

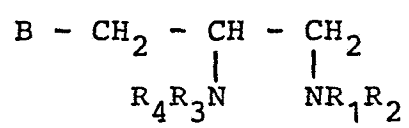

II

oder einem Salz der Verbindung II mit einem physiologisch verträglichen Gegenion oder einem Säureadditionssalz der Verbindung II umsetzt, wobei die Reste $R_1$, $R_2$, $R_3$, $R_4$ und B die angegebenen Bedeutungen haben, gegebenenfalls in Hydroxygruppen oder Aminogruppen des Restes B durch Acylierung eine oder mehrere $C_1$-$C_6$-Alkanoylgruppen, $C_1$-$C_6$-Alkansulfonylgruppen oder Benzoylgruppen einführt, gegebenenfalls vorhandene Amino-

...

gruppen des Restes B zu Nitrogruppen oxydiert, und gegebenenfalls in einer erhaltenen Verbindung der Formel I den Rest X beziehungsweise die Reste X gegen andere physiologisch verträgliche Anionen austauscht und/oder gegebenenfalls erhaltene Verbindungen in die Salze mit physiologisch verträglichen Anionen oder Kationen überführt.

3. Arzneimittel, enthaltend eine Verbindung der allgemeinen Formel I neben üblichen Träger- und/oder Verdünnungs- beziehungsweise Hilfsstoffen.

4. Verfahren zur Herstellung eines Arzneimittels, dadurch gekennzeichnet, daß eine Verbindung der allgemeinen Formel I mit gebräuchlichen pharmazeutischen Trägerstoffen oder Verdünnungsmitteln beziehungsweise sonstigen Hilfsstoffen zu pharmazeutischen Zubereitungen verarbeitet beziehungsweise in eine therapeutisch anwendbare Form gebracht wird.

5. Verwendung von Verbindungen der allgemeinen Formel I zur Herstellung von Arzneimitteln.

...

Europäisches Patentamt

# EUROPÄISCHER RECHERCHENBERICHT

Nummer der Anmeldung 0193083

EP 86 10 2092

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP-A-0 055 300 (OTSUKA CHEMICAL)<br>* Ansprüche *<br>----- | 1,3-5 | C 07 F 15/00<br>A 61 K 31/28 |
| | | | RECHERCHIERTE SACHGEBIETE (Int. Cl.4) |
| | | | C 07 F 15/00<br>A 61 K 31/00 |

Der vorliegende Recherchenbericht wurde für alle Patentansprüche erstellt.

| Recherchenort | Abschlußdatum der Recherche | Prüfer |
|---|---|---|
| DEN HAAG | 26-05-1986 | BESLIER L.M. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82